(19) Europäisches Patentamt
European Patent Office
Office européen des brevets

(11) EP 4 745 175 A1

(12) EUROPEAN PATENT APPLICATION

(43) Date of publication:
**20.05.2026 Bulletin 2026/21**

(21) Application number: **24315523.1**

(22) Date of filing: **15.11.2024**

(51) International Patent Classification (IPC):
***C08G 18/42*** *(2006.01)* ***C08G 18/81*** *(2006.01)*
***C08G 63/91*** *(2006.01)*

(52) Cooperative Patent Classification (CPC):
(C-Sets available)
**C08G 18/4244; A61L 24/0042; A61L 24/046; B33Y 70/00; B33Y 80/00; C08G 18/4615; C08G 18/8116; C08G 63/672; C08G 63/6856; C08G 63/916;** C08G 2190/00 (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Tissium SA**
**75012 Paris (FR)**

(72) Inventors:
- **Gerbouin, Prune**
  **75020 PARIS (FR)**
- **Bernardi, Sarah**
  **91300 MASSY (FR)**
- **Legros, Camille**
  **75012 PARIS (FR)**
- **Maie E Silva, João Reina**
  **4150-175 PORTO (PT)**
- **Gaillot, Cassandre**
  **13002 MARSEILLE (FR)**
- **Rhoné, Benoît**
  **78200 MANTES LA JOLIE (FR)**

(74) Representative: **Opilex**
**32, rue Victor Lagrange**
**69007 Lyon (FR)**

Remarks:
Claims 16 to 26 are deemed to be abandoned due to non-payment of the claims fees (Rule 45(3) EPC).

# (54) COMPOSITION COMPRISING PRE-POLYMER WITH IMPROVED MECHANICAL PROPERTIES

(57) A composition comprising a pre-polymer having a polymeric backbone derived from a diol and a diacid is disclosed. The composition may be used in a method of joining or sealing tissue, or for joining a medical device to the surface of a tissue.

EP 4 745 175 A1

(52) Cooperative Patent Classification (CPC): (Cont.)

C-Sets
**A61L 24/046, C08L 71/02**

## Description

### FIELD OF INVENTION

The present invention relates to a composition comprising a pre-polymer, a method of manufacturing the composition, a method of curing the composition, a cured composition obtainable therefrom, uses of the composition and methods of using the composition.

### BACKGROUND OF THE INVENTION

Polymeric tissue sealants and adhesives provide materials for tissue repair and are widely used in a variety of medical settings ranging from minor to life-threatening tissue injuries. They are for example an alternative to sutures and staples to close and seal wounds or incisions. These materials are advantageous because of their ease of use, short application time and minimal tissue damage, making them suitable for minimally invasive procedures. An ideal tissue adhesive should have the following properties: strong adhesion to tissue, especially under wet conditions; an ability to form a watertight seal; biodegradability; mechanical compliance with the underlying substrate (that is, the tissue); low cytotoxicity and minimal inflammatory response. Despite their numerous current applications, tissue adhesives still face several limitations and unresolved challenges (e.g., weak adhesion strength, poor mechanical properties and safety issues) that limit their use, leaving ample room for improvements.

Current clinically-available adhesives, such as medical grade cyanoacrylate (CA) or fibrin sealant, are easily washed out or cured under dynamic wet conditions, are toxic and cannot be used internally, and/or exhibit weak adhesive properties such that they cannot withstand the forces inside the cardiac chambers and major blood vessels. Also, many of these adhesives exhibit activation properties that make fine adjustments or repositioning of the devices very difficult. Moreover, many adhesives under development achieve tissue adhesion only through chemical reaction with functional groups at the tissue surface, and thus become ineffective in the presence of blood.

Alternatives to cyanoacrylate have been explored. US 8,143,042 describes biodegradable elastomers prepared by crosslinking a prepolymer containing crosslinkable functional groups, such as acrylate groups. It also discloses that it is desirable to increase the number of free hydroxy groups on the polymer in order to increase the stickiness of the polymer. Increasing the number of hydroxy groups in the backbone also leads to enhanced solubility in physiologic solutions. This suggests that the primary mechanism of adhesion of the polymer is chemical interactions between functional groups, for example free hydroxy groups on the polymer and the tissue to which it is applied. However, this type of chemical interaction becomes ineffective in the presence of body fluids, especially blood, as shown in Artzi et al., Adv. Mater. 21, 3399-3403 (2009).

Elastomeric crosslinked polyesters are disclosed in US 2013/0231412. Biodegradable polymers are disclosed in US 7,722,894. Adhesive articles are disclosed in WO 2009/067482 and WO 2014/190302 A1. Blood resistant surgical glue is described in Lang et al. "A Blood-Resistant Surgical Glue for Minimally Invasive Repair of Vessels and Heart Defects," Sci. Transl. Med., 8 January 2014: Vol. 6, Issue 218, p. 218ra6 and WO 2014/190302 Al.

Patel et al (Biomaterials, 34(16), 3970-3983) disclose highly elastomeric poly(glycerol-sebacate)-co-poly(ethylene glycol) amphiphilic block copolymers. A range of PGS-co-PEG polymer from mechanically stiff to elastomeric soft was synthesized.

WO 2021/078962 discloses polyglycerol sebacate (PGS) polymers that have been acrylated by reaction with acryloyl chloride and a proportion of the acrylated moities are then aminated by reaction with diethylamine. The arylated and aminated pre-polymer is acidified to provide positively charged nitrogen groups. An improvement in adhesion is observed as the zeta potential of the pre-polymer compositions increases.

Moreover, despite current efforts in developing these elastomeric polymers and although some of them adhere to target tissue, it has been shown that they present low resistance to cracking and breaking when used with soft tissue, especially during repeated and prolonged tissue deformation.

### SUMMARY OF THE INVENTION

The invention provides an improved and commercially viable pre-polymer that can be readily applied to the desired site, is biocompatible (non-toxic), and exhibits strong forces once cured/crosslinked leading to improved tissue sealant/fixation.

The pre-polymer remains in place at the desired site prior to curing, even in the presence of bodily fluids, such as blood.

The pre-polymer, and more particularly the polymer obtained by curing the pre-polymer, exhibits improved mechanical properties compared to previously disclosed pre-polymers/polymers. In particular, the polymer may exhibit mechanical properties approaching those of natural tissue, e.g. soft tissue.

More particularly, the invention provides a pre-polymer having a polymeric backbone derived from a diol and a diacid, wherein the pre-polymer comprises terminal groups derived from a polyol,
and wherein the pre-polymer comprises activated groups and optionally further comprises functionalized groups, wherein the functionalized groups include a positively charged heteroatom.
The present invention further provides a composition comprising the pre-polymer according to the invention.
The present invention also provides methods for preparing the pre-polymer and composition of the present invention.
The present invention further provides a method of curing the composition according to the present invention, comprising curing the composition with a stimulus, for example light in the presence of a photo-initiator.
The present invention also provides a cured composition obtainable by the curing method according to the present invention. Desirably said cured composition can fix strongly to a surface or can fix one surface to another.
The present invention further provides methods of use and use of the composition according to the present invention for sealing tissue, for joining tissues or for fixing a medical device to tissue.
The present invention further provides methods of use and use of the composition according to the present invention for preparing printing resin (e.g. 3D printing resin) and for producing shaped objects by a variety of techniques known in the art, including 3D printing. The invention further provides printed (e.g. 3D printed) shaped objects obtained by using the composition of the invention.
The inventors have found that, compared to known compositions, the present invention offers advantages which are not found in the prior art.

## BRIEF DESCRIPTION OF THE FIGURES

Figures 1A and 1B illustrate a synthetic method according to an embodiment of the invention.

Figures 2A and 2B illustrate another synthetic method according to an embodiment of the invention.

Figure 3 shows the variation of carboxylic acid content of pre-polymers with molecular weight.

Figure 4 shows the variation of hydroxy content of pre-polymers with molecular weight.

Figures 5A, 5B, 5C, 5D illustrate the standard stress/strain curve obtained through the assessment of mechanical properties with several example of of cured/crosslinked polymers of the Invention relative to the comparative polymer.

Figure 6 illustrates modularity of the synthesis method of the invention.

Figures 7A and 7B show degradation properties for a range of cured/crosslinked polymers.

Figures 8A and 8B illustrate fixation strength of examples of cured/crosslinked polymer of the Invention.

Figures 9A and 9B illustrate fixation strength of examples of cured/crosslinked polymer produced with different functionalization methods.

Figure 10 illustrates mechanical properties after incubation of the cured/crosslinked polymer in a liquid.

Figure 11 illustrates resistance to bending of the cured/crosslinked polymer.

## DETAILED DESCRIPTION OF THE INVENTION

Polymeric backbone

The polymeric backbone of the pre-polymer is derived from a diol and a diacid. Other components (including additional diols and/or additional diacids) may be present in the polymeric backbone, but the backbone is primarily derived from the diol and the diacid.
In one embodiment of the invention, the diol is a polyether. A polyether is a polymer wherein the repeating unit contains a carbon-oxygen bond. The polyether may be polyethylene glycol:

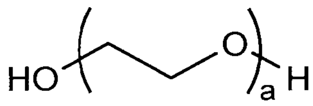

a is an integer greater than 1 and will be a range of different values for a polydisperse polyethylene glycol. The molecular weight (i.e. number average molecular weight, $M_n$) of the polyethylene glycol is preferably greater than 150 g/mol and more preferably greater than 200 g/mol. The molecular weight (i.e. number average molecular weight, $M_n$) of the polyethylene glycol is preferably less than 1500 g/mol, more preferably less than 1000 g/mol, even more preferably less than 700 g/mol. Commercial polyethylene glycol grades that would be suitable for use in the present invention include PEG200, PEG400 and PEG600.

In another embodiment of the invention, the diol is an aliphatic diol wherein the hydroxy groups are on the terminal carbon atoms:

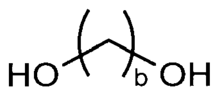

b is an integer of 4 or more, preferably an integer of 6 or more. Diols that could be used include 1,8-octanediol; 1,7-heptanediol; 1,6-hexanediol; 1,5-pentanediol; and 1,4-butanediol.

In another embodiment of the invention, the diol is an aliphatic diol wherein the hydroxy groups are not on the terminal carbon atoms. A commercial aliphatic diol that would be suitable for use in the present invention is 2-butyl-2-ethyl-1,3-propanediol.

In another embodiment of the invention the diol is polycaprolactone diol.

Exemplary diacids include, but are not limited to, succinic acid (4 carbons), glutaric acid (5 carbons), adipic acid (6 carbons), pimelic acid (7 carbons), suberic acid (8 carbons), azelaic acid (9 carbons), and sebacic acid (10 carbons), wherein each diacid may be substituted or unsubstituted. Exemplary long chain diacids include diacids having more than 10, more than 15, more than 20, and more than 25 carbon atoms. Polyethylene glycol diacid, e.g. PEG 600 diacid, could be used. Non-aliphatic diacids can also be used. For example, versions of the above diacids having one or more double bonds can be used to produce polyol-diacid copolymers. Preferably the polyacid is substituted or unsubstituted sebacic acid. According to another special embodiment, the polyacid is selected in the group consisting in succinic acid, glutaric acid and adipic acid.

The term "substituted" has its usual meaning in chemical nomenclature and is used to describe a chemical compound in which a hydrogen on the primary carbon chain has been replaced with a substituent such as alkyl, aryl, carboxylic acid, ester, amide, amine, urethane, ether, or carbonyl.

In an embodiment of the invention the polymeric backbone is derived from polyethylene glycol and sebacic acid.

In another embodiment of the invention the polymeric backbone is derived from polyethylene glycol and succinic acid, glutaric acid or adipic acid.

According to a preferred embodiment, the polymeric backbone of the pre-polymer of the invention is linear, or mostly linear.

According to a preferred embodiment, the polymeric backbone of the pre-polymer is of the general formula (I):

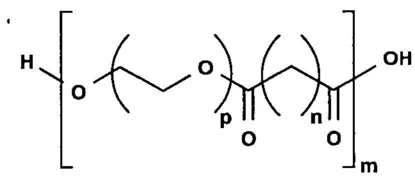

(I)

wherein n, p and m each independently represent an integer greater than 1. If the backbone is made using a polydisperse polymeric diol then the value of p will vary between different monomers in the pre-polymer of general formula (I).

<u>Terminal Groups</u>

Polymerization of the diol and the diacid provides a polymeric backbone with 50% carboxylic acid end chains and 50% hydroxyl end chains:

HO-Polymeric backbone-OH

HOOC-Polymeric backbone-COOH

HO-Polymeric backbone-COOH

HOOC-Polymeric backbone-OH

In a second stage, reaction of at least part of these carboxylic acid end chains with a polyol provides the terminal groups derived from a polyol.

The polyol preferably has 3, 4, 5 or 6 hydroxy groups. The polyol is preferably not the same as the diol that is used to provide the polymeric backbone. Suitable triols include glycerol and trimethylolpropane ethoxylate. Suitable tetraols include di(trimethylolpropane). Suitable higher polyols include xylitol.

In an embodiment of the invention, the polyol is serinol. Serinol is a diol but additionally has a primary amine functional group.

According to an embodiment, in the pre-polymer of the invention, the polymeric backbone is derived from polyethylene glycol and sebacic acid, and at least part of the terminal groups are derived from glycerol. Reaction of glycerol with the carboxylic acid end groups of the polymeric backbone provides mostly terminal groups having two hydroxyl groups. These hydroxyl groups may be further reacted.

Alternatively, sebacic acid can be replaced with succinic acid, glutaric acid or adipic acid.

According to a special embodiment, the polymeric backbone having terminal groups derived from a polyol is of the general formula (II):

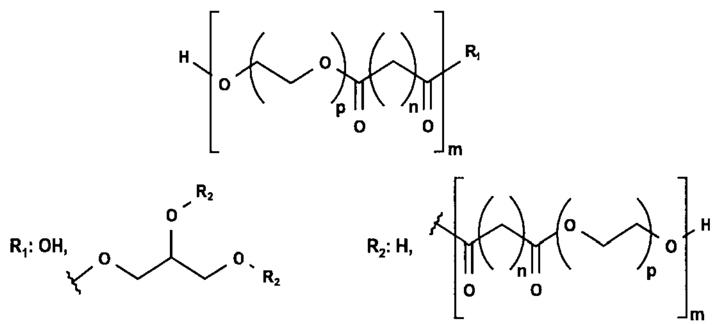

(II)

wherein n, p and m each independently represent an integer greater than 1.

<u>Activated Groups</u>

The pre-polymer of the invention comprises activated groups.

The activated groups are functional groups that can react or be reacted to form crosslinks.

Suitable functional groups to be activated on the pre-polymer (including both the polymeric backbone and terminal groups) include hydroxy groups, carboxylic acid groups, amines and combination thereof.

In an embodiment, the activated group is or contains a vinyl group. According to the present invention, vinyl groups contain the following structure $-CR_g=CR_hR_i$, wherein $R_g$, $R_h$, $R_i$ are independently from one another, selected from the group consisting of H, alkyl such as methyl or ethyl, aryl such as phenyl, substituted alkyl, substituted aryl, carboxylic acid, ester, amide, amine, urethane, ether, and carbonyl.

In one embodiment, the activated group is or contains an acrylate or a methacrylate group. According to the present invention, acrylate groups may contain the following group: $-C(=O)-CR_g=CR_hR_i$, wherein $R_g$, $R_h$, $R_i$ are independently from one another, selected from the group consisting of H, alkyl such as methyl or ethyl, aryl such as phenyl, substituted alkyl, substituted aryl, carboxylic acid, ester, amide, amine, urethane, ether, and carbonyl.

According to another embodiment, acrylate groups may contain the following group: $-C(=O)NR_d-(CR_eR_f)_j-O-C(=O)-CR_g=CR_hR_i$, wherein $R_d$, $R_e$, $R_f$, $R_g$, $R_h$ and $R_i$ are independently from one another, selected from the group consisting of H, alkyl, such as methyl or ethyl, aryl, such as phenyl, substituted alkyl, substituted aryl, carboxylic acid, ester,

amide, amine, urethane, ether, and carbonyl; and j is an integer greater than 1 (e.g. j is 2). According to an embodiment, the activated pre-polymer contains a mixture of different acrylate groups.

Preferably, all or part of the acrylate groups containing the $-C(=O)-CR_g=CR_hR_i$ group are such that $R_g$, $R_h$ and $R_i$ are H; or such that $R_g$ is $CH_3$, $R_h$ and $R_i$ are H; or such that $R_g$ and $R_h$ are H and $R_i$ is $CH_3$; or such that $R_g$ and $R_h$ are H and $R_i$ is phenyl.

Preferably, all or part of the acrylate groups containing the $C(=O)NR_d-(CR_eR_f)_j-O-C(=O)-CR_g=CR_hR_i$ group are such that $R_d$, $R_e$, $R_f$, $R_g$, $R_h$ and $R_i$ are H, and j is 2; or such that $R_g$ is $CH_3$, $R_d$, $R_e$, $R_f$, $R_h$ and $R_i$ are H, and j is 2; or such that $R_d$, $R_e$, $R_f$, $R_g$ and $R_h$ are H, $R_i$ is $CH_3$, and j is 2; or such that $R_d$, $R_e$, $R_f$, $R_g$ and $R_h$ are H, $R_i$ is phenyl and n is 2.

The amount of activation (e.g. acrylation) is suitably measured by a technique such as $^1H$ NMR. The activation concentration is suitably characterized as an amount (in mmol) based upon the mass (in g) of the pre-polymer.

The preferred concentration of activation may vary depending upon the ultimate application of the pre-polymer composition. The amount of activation is preferably between 0.03 mmol/g and 4 mmol/g, more preferably between 0.05 mmol/g and 1 mmol/g and most preferably between 0.2 mmol/g and 1.5 mmol/g. For fixation applications the amount of activation may be, e.g. between 0.2 mmol/g and 0.8 mmol/g. For 3D printing applications the amount of activation may be, e.g. between 0.4 mmol/g and 1.5mmol/g.

Functionalized Groups

The pre-polymer of the invention optionally comprises functionalized groups. In a preferred embodiment of the invention, the composition of the invention comprises activated groups and functionalized groups. The functionalized groups include a positively charged heteroatom.

The positively charged heteroatom may be derived from any element other than carbon or hydrogen. Preferred positively charged heteroatoms are nitrogen, phosphorus and sulfur.

Most preferably, the positively charged heteroatom is a positively charged nitrogen atom.

The amount of functionalized groups is suitably measured by a technique such as $^1H$ NMR. The degree of functionalization is suitably characterized as an amount (in mmol) based upon the mass (in g) of the pre-polymer.

In an embodiment of the invention the amount of functionalization is between 0 mmol/g and 4 mmol/g, preferably between 0.1 mmol/g and 1 mmol/g.

The ratio of activated groups to functionalized groups is suitably in the range of from 1:10 to 10:1, preferably from 1:5 to 5:1, and most preferably is from 2:1 to 1:2.

The functionalized groups including a positively charged nitrogen atom are preferably of the general formula (III):

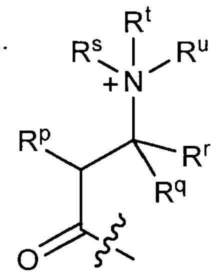

(III)

wherein $R^p$, $R^q$, $R^r$, $R^s$, $R^t$ and $R^u$ are independently selected from H, alkyl, alkenyl and aryl. Preferably at least one of $R^s$, $R^t$ and $R^u$ is H.

Alkyl groups for $R^p$, $R^q$, $R^r$, $R^s$, $R^t$ and $R^u$ are suitably selected from the group consisting of straight chain alkyl groups (e.g., methyl, ethyl, propyl, butyl, pentyl, hexyl, heptyl, octyl, nonyl, decyl, etc.) or branched-chain alkyl groups (isopropyl, tert-butyl, isobutyl, etc.), cycloalkyl (alicyclic) groups (cyclopropyl, cyclopentyl, cyclohexyl, cycloheptyl, cyclooctyl) or alkyl-substituted cycloalkyl groups. Preferably any alkyl groups are $C_{1-8}$ alkyl groups, more preferably $C_{1-4}$ alkyl groups and most preferably methyl or ethyl groups.

Alkenyl groups for $R^p$, $R^q$, $R^r$, $R^s$, $R^t$ and $R^u$ are suitably selected from the groups consisting of straight-chain alkenyl groups (e:g., ethenyl, propenyl, butenyl, pentenyl, hexenyl, heptenyl, octenyl, nonenyl, decenyl, etc.) or branched-chain alkenyl groups, cycloalkenyl (alicyclic) groups (cyclopropenyl, cyclopentenyl, cyclohexenyl, cycloheptenyl, cyclooctenyl), alkyl or alkenyl substituted cycloalkenyl groups, and cycloalkyl or cycloalkenyl substituted alkenyl groups. Preferably any alkenyl groups are $C_{2-8}$ alkenyl groups.

Aryl groups for $R^p$, $R^q$, $R^r$, $R^s$, $R^t$ and $R^u$ are suitably selected from the groups consisting of 5- and 6-membered

single-ring aromatic groups, as well as multicyclic aryl groups, such as tricyclic or bicyclic (e.g., naphthalene, anthracene, phenanthrene, etc.). Aryl groups can also be fused or bridged with, e.g., alicyclic or heterocyclic rings which are not aromatic so as to form, e.g., a polycycle.

Preferably $R^p$ is hydrogen. Preferably $R^q$ is hydrogen. Preferably $R^r$ is hydrogen.

Preferably one, two or three of $R^s$, $R^t$ and $R^u$ are hydrogen. Most preferably one of $R^s$, $R^t$ and $R^u$ is hydrogen. In another embodiment, $R^s$, $R^t$ and $R^u$ are not hydrogen.

Alternatively, the functionalized groups including a positively charged nitrogen atom are preferably of the general formula (IV):

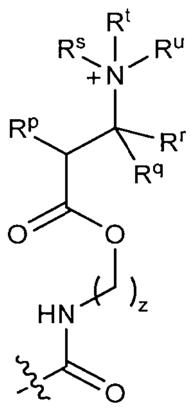

(IV)

wherein $R^p$, $R^q$, $R^r$, $R^s$, $R^t$ and $R^u$ are as defined above for groups of formula (III), and
z represents an integer equal to or greater than 1, preferably from 1 to 4.

According to a preferred embodiment, the functionalized group of the general formula (IV) is:

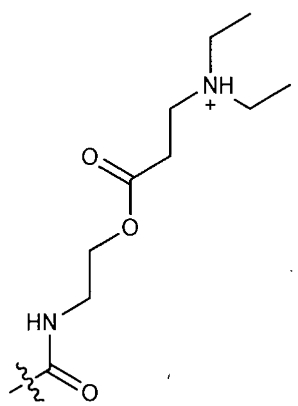

Alternative functional groups are as follows:

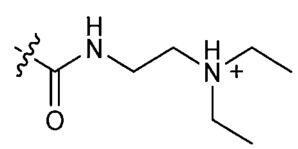 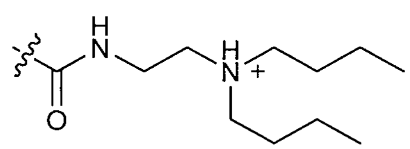 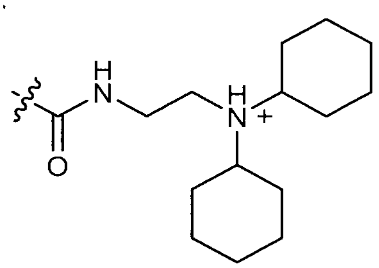

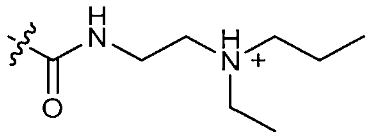
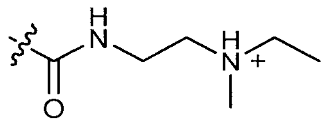
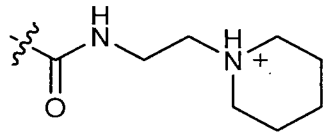
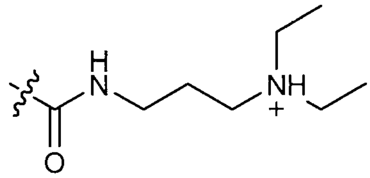
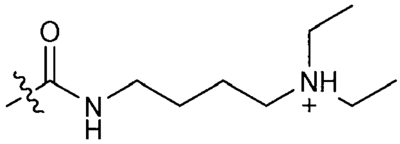
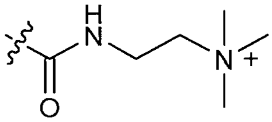
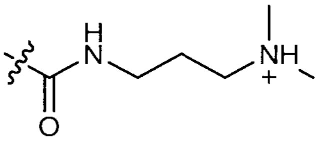
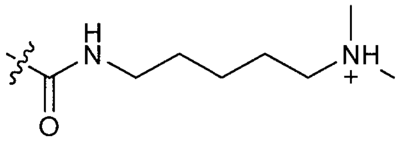
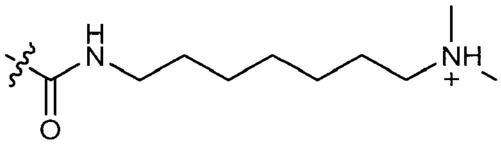
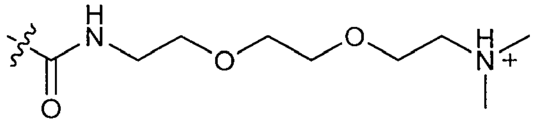
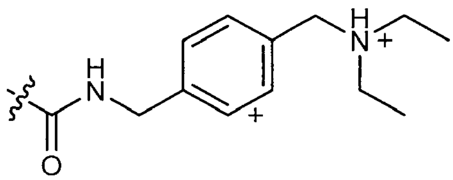

Pre-polymers

The number average molecular weight of the pre-polymer ($M_n$) is suitably from about 800 to about 30,000 g/mol, preferably from about 1,000 to about 25,000 g/mol, more preferably from about 1,200 to about 20,000 g/mol.

The number average molecular weight of the pre-polymer ($M_n$) may be measured by Gel Permeation Chromatography equipped with a refractive index and polystyrene calibration standards

The term "about" as used herein means within 10%, preferably within 8%, and more preferably within 5% of a given value or range. According to a specific embodiment, "about X" means X, when X refers to the value or range.

The pre-polymer may have a polydispersity, measured by Gel Permeation Chromatography equipped with a refractive index, below 20.0, more preferably below 10.0, more preferably below 5.0, and even more preferably below 3

The molar ratios of the diol to the diacid in the pre-polymer backbone are suitably in the range of about 0.5:1 to about 1.5:1, preferably in the range of about 0.9:1.1 to about 1.1:0.9 and most preferably about 1:1.

According to a special embodiment, the pre-polymer is as shown in the final product in Figures 1A and 1B. According to another special embodiment, the pre-polymer is as shown in the final product in Figures 2A and 2B.

Composition

The invention further provides a composition comprising a pre-polymer as described above.

The composition according to the present invention can be manufactured in the presence and/or mixed with a coloring agent. Preferred examples of coloring agents are the ones recommended by the FDA for use in medical devices, pharmaceutical products or cosmetics.

Similarly, the composition can further comprise stabilizers, for example 4-methoxyphenol (MEHQ), N-Phenyl-2-naphthylamine (PBN), phenothiazine (PTZ) and/or 5,5-Dimethyl-1-pyrroline N-oxide (DMPO).

The pre-polymer of the composition can be further reacted with one or more additional materials to modify the crosslinks between the polymer chains. For example, prior to or during curing/crosslinking (curing and crosslinking are

synonyms), one or more hydrogel or other oligomeric or monomeric or polymeric precursors (e.g., precursors that may be modified to contain acrylate groups) such as poly(ethylene glycol), dextran, chitosan, hyaluronic acid, alginate, other acrylate based precursors including, for example, acrylic acid, butyl acrylate, 2-ethylhexyl acrylate, methyl acrylate, ethyl acrylate, acrylonitrile, n-butanol, methyl methacrylate, acrylic anhydride, metahcrylic anhydride and TMPTA, trimethylol propane trimethacrylate, pentaerythritol trimethacrylate, pentaerythritol tetramethacrylate, ethylene glycol dimethacrylate. dipentaerythritol penta acrylate, Bis-GMA (Bis phenol A glycidal methacrylate) and TEGDMA (tri-ethylene, glycol dimethacrylate), sucrose acrylate; other thiol based precursors (monomeric or polymeric); other epoxy based precursors; and combinations thereof, can be reacted with the acrylated pre-polymer.

The composition according to the present invention can be a surgical composition and is suitably used as a tissue sealant, for joining tissues or for fixing a medical device to the surface of tissue. The composition suitably has flow characteristics such that it can be applied to the desired area through a syringe or catheter but is sufficiently viscous to remain in place at the site of application without being washed away by bodily fluids, such as water and/or blood.

Preferably, the viscosity of the composition is 500 to 100,000 cP, more preferably 1,000 to 50,000 cP, even more preferably 2,000 to 40,000 cP and most preferably 2,500 to 25,000 cP. Viscosity analysis is performed using a Brookfield DV-II + Pro viscosimeter with a 2.2mL chamber and SC4-14 spindle, the speed during the analysis is varied from 5 to 80 rpm. The above-mentioned viscosity is present in the relevant temperature range for medical application i.e. room temperature up to 40°C, preferably 37°C.

The composition of the invention may be incubated in bodily fluids, such as blood, prior to curing, without a substantial decrease in fixing strength when cured.

The composition of the invention is suitably stable in bodily fluids, such as blood. More particularly, the composition of the invention suitably does not spontaneously crosslink in bodily fluids absent the presence of an intentionally applied stimulus such as light, for example UV light, visible light, heat, or chemical initiator to initiate crosslinking.

The composition can be cured using a free radical initiated reaction, such as, for example, by photo-initiated polymerization, thermally-initiated polymerization, and redox initiated polymerization.

Preferably, the composition of the invention is irradiated with light, for example ultraviolet (UV) light and preferably with visible light (typically blue light or green light). Preferably the composition of the invention is irradiated with light in the presence of a photoinitiator to facilitate the reaction. Accordingly, in a preferred embodiment, the composition of the invention contains a photoinitiator to facilitate the reaction . Examples of suitable photoinitiators include, but are not limited to: 2-dimethoxy-2-phenyl-acetophenone, 2-hydroxy-1-[4-(hydroxyethoxy)phenyl]-2-methyl-1-propanone (Irgacure 2959), 1-hydroxycyclohexyl-1-phenyl ketone (Irgacure 184), 2-hydroxy-2-methyl-1-phenyl-1-propanone (Darocur 1173), 2-benzyl-2-(dimehylamino)-1-[4-morpholinyl) phenyl]-1-butanone (Irgacure 369), methylbenzoylformate (Darocur MBF), oxy-phenyl-acetic acid-2-[2-oxo-2-phenyl-acetoxy-ethoxy]-ethyl ester (Irgacure 754), 2-methyl-1-[4-(methylthio) phenyl]-2-(4-morpholinyl)-1-propanone (Irgacure 907), diphenyl(2,4,6-trimethylbenzoyl)-phosphine oxide (e.g. Darocur TPO, Omnirad TPO), Ethyl(2,4,6-Trimethylbenzoyl)-phenyl phosphinate (Speedcure TPO-L, Omnirad TPO-L), phosphine oxide, phenyl bis(2,4,6-trimethyl benzoyl) (Irgacure 819), eosin Y disodium salt, N-Vinyl-2-Pyrrolidone (NVP) and triethanolamine, and camphorquinone, and combinations thereof.

In applications of the composition involving *in vivo* photopolymerization and other medical applications, the use of cytocompatible photoinitiators is preferred and may be required by regulatory agencies. Photoinitiator Irgacure 2959 or Omnirad TPO may be used, which causes minimal cytotoxicity (cell death) over a broad range of mammalian cell types and species.

In order for the photopolymerization to occur, the composition (and the substrate to which the composition is applied, if applicable) is preferably sufficiently transparent to the light.

In applications when the composition is cured *in vivo,* the temperature at which curing occurs is preferably controlled as not damage the tissue on which the composition has been applied. Preferably, the composition is not heated above 45°C during irradiation, more preferably not above 37°C, and even more preferably not above 25°C.

In addition to photochemical crosslinking, the composition can be cured thermally, by Mitsunobu-type reaction, by redox-pair initiated polymerization for example benzoyl peroxide, N,N,-dimethyl-p-toluidine, ammonium persulfate, or tetramethylenediamine (TEMED), and by a Michael-type addition reaction using a bifunctional sulfhydryl compound.

In one embodiment, a composition according to the invention may comprise the redox initiator system comprising:

- at least one oxidant selected in the group consisting in APS (Ammonium persulfate), KPS (Potassium persulfate) or BPO (Benzoyl peroxide);

- at least one reducing agent selected in the group of TMA (4-N,N Trimethylaniline), N,N-Bis(2-hydroxyethyl)-p-toluidine, N,N-Dimethylaniline, N,N-Diethylaniline, sodium p-toluenesulfonate, N-Methyl-N-(2-hydroxyethyl)-p-toluidine, MHPT (N-(2-Hydroxyethyl)-N-methyl-para-toluidine) and Phosphine (Diphenylphosphinostryrene or triphenylphosphine);

- and at least one radical scavenger selected in the group consisting of Tempol and or 4-methoxyphenol.

According to special embodiment, a redox composition may comprise 0.1 to 5 wt% of a reducing agent, e.g., 4-N,N Trimethylaniline, N,N-Bis(2-hydroxyethyl)-p-toluidine, N,N-Dimethylaniline, N,N-Diethylaniline, sodium p-toluenesulfonate or N-Methyl-N-(2-hydroxyethyl)-p-toluidine; 0 to 5 wt% of an oxygen inhibitor, e.g., 4-(Diphenylphosphino) styrene or triphenylphosphine; 0.005 to 0.5 wt% of a working time agent, e.g., Tempol or 4-methoxyphenol; and 0.1 to 10 wt% of an oxidant, e.g., ammonium persulfate, potassium persulfate or benzoyl peroxide.

Pre-polymer/polymer performances

Upon polymerization, the pre-polymer forms a crosslinked network with improved properties and exhibits significant fixation strength even in the presence of blood and other bodily fluids. The cured polymer is preferably sufficiently elastic to resist movement of the underlying tissue, e.g. soft tissue, for example contractions of the heart and blood vessels, or expansion of the gastrointestinal tract or bladder. The cured polymer can provide a seal, preventing the leakage of fluids or gas. The cured polymer is preferably biodegradable and biocompatible, causing minimal inflammatory response. The cured polymer is preferably elastomeric.

Biodegradability can be evaluated *in vitro,* such as in phosphate buffered saline (PBS) or in acidic or alkaline conditions. Biodegradability can also be evaluated *in vivo,* such as in an animal, for example mice, rats, dogs, pigs or humans. The rate of degradation can be evaluated by measuring the loss of mass of the polymer over time *in vitro* or *in vivo.*

The cured composition, alone or coated on a patch or tissue suitably exhibits a 90° pull off fixation strength of at least 0.5 $N/cm^2$, preferably at least 1 $N/cm^2$ and even more preferably at least 2 $N/cm^2$, for example 1.5 $N/cm^2$ to 2 $N/cm^2$, but preferably greater than 5 $N/cm^2$, for example up to 6 $N/cm^2$ or 7 $N/cm^2$ or greater. Pull off fixation strength refers to the fixation value obtained by attaching an article or sample to wet tissue, such as epicardial surface of cardiac tissue or blood vessels immobilized on a flat substrate, such as a metallic stub. The 90° pull off fixation test determines the greatest perpendicular force (in tension) that a surface area can bear before detachment (N. Lang et al., Sci. Transl. Med., 2014, 6, 218ra6).

According to preferred embodiment, the composition of the invention is cured by light and in presence of a photo initiator and the cured composition exhibits a 90° pull off fixation strength of at least 0.5 $N/cm^2$, preferably at least 1 $N/cm^2$ and even more preferably at least 2 $N/cm^2$, for example 1.5 $N/cm^2$ to 2 $N/cm^2$, but preferably greater than 5 $N/cm^2$, for example up to 6 $N/cm^2$ or 7 $N/cm^2$ or greater.

According to preferred embodiment, the composition of the invention is able after curing to fix strongly to a surface or to fix one surface to another.

Method of preparation

The method for preparing the pre-polymer of the present invention, comprises several required steps, which may accommodate several variations. According to a preferred embodiment, said method comprises the steps of:

i) polymerization of the diol and the diacid to provide the polymeric backbone;

ii) reaction of the polymeric backbone with the polyol to provide additional hydroxy terminal groups;

iii) activation to provide activated groups; and

iv) optionally, functionalization with functionalized groups, wherein the functionalized groups include a positively charged heteroatom.

In a preferred embodiment, step (iii) takes place after step (ii) such that the additional hydroxy terminal groups may be activated. If step (iv) is present in the method, then this may be before or after step (iii).

The conditions for polymerization may include a temperature range of 100 to 140°C, preferably 120 to 130°C, an inert atmosphere, preferably comprising nitrogen, and under vacuum.

The product of step (i) has terminal carboxylic acid groups. In step (ii) these groups are reacted with a polyol, thereby increasing the number of hydroxy groups in the polymer, e.g. with glycerol:

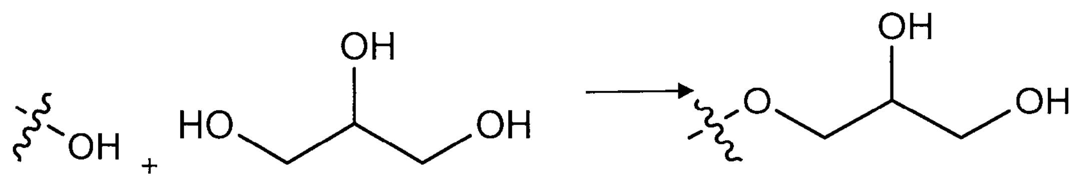

It is possible that multiple hydroxy groups on the polyol will react, but desirably the reaction is controlled such that each polyol reacts once with the polymer, thereby increasing the amount of terminal hydroxy groups on the polymer.

In an embodiment, the reaction with polyol is carried out using Steglich esterification. For example, in one method the polymer, the polyol and N,N-dimethylpyridin-4-amine (DMAP) are added to tetrahydrofuran (THF) and the mixture is stirred at room temperature until a clear homogeneous mixture is obtained. N,N'-dicyclohexylcarbodiimide (DCC) is added and the mixture is stirred at room temperature. A precipitate appears during the reaction. The solution is filtered and concentrated under reduced pressure. The residue is resuspended in dichloromethane (DCM). The organic layer is collected, washed, dried, filtered and concentrated.

In another embodiment, the reaction with polyol is carried out using polycondensation. For example, in one method the polymer and the polyol are placed in a flask and heated. No catalyst is added. After complete melting of the mixture, the flask is placed under dynamic vacuum (vacuum set at 15 mBar) and left to react. After the reaction, the solution is dissolved in DCM and washed. The organic phase is collected, dried, filtered and concentrated.

The activation in step iii) is suitably achieved by partial acrylation of the terminal hydroxy groups.

In an embodiment of the invention acrylation is achieved using an isocyanate acrylate compound, such as 2-isocyanatoethylmethacrylate. For example, in one method the polymer with terminal hydroxy groups is mixed with 4-methoxyphenol (MEHQ) in ethyl acetate (EtOAc). The mixture is heated and stirred and then 2-isocyanatoethylmethacrylate is added, e.g.

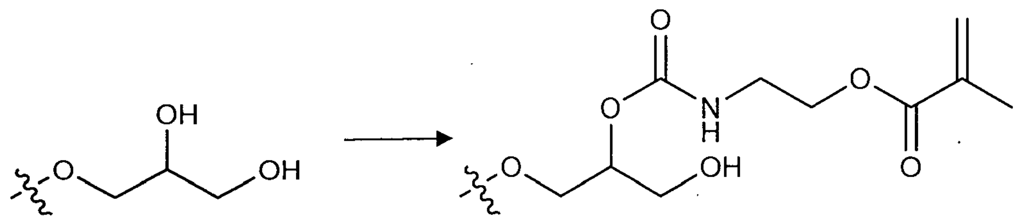

In another embodiment of the invention acrylation is achieved using a compound such as acryloyl chloride, e.g.

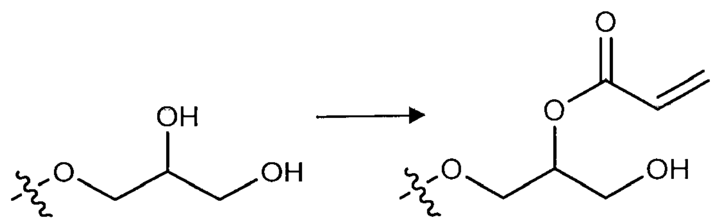

In an embodiment of the invention, functionalization in step (iv) is suitably achieved by a sequence of acrylation, amination and acidification.

In an embodiment of the invention, functionalization in step (iv) is achieved using an isocyanate acrylate compound, such as 2-isocyanatoethyl-acrylate, followed by reaction with diethylamine and acidification with acetic acid, e.g.

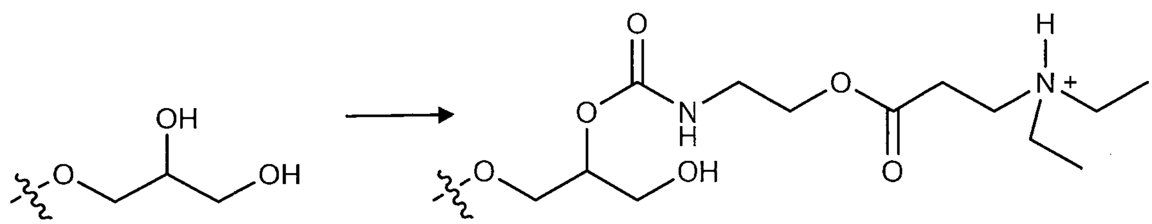

At least one additive may be added to the composition obtained at step (iv). In a preferred embodiment, said

additive is selected from the group consisting of photoinitiators, radical inhibitors, and dyes.

According to a preferred embodiment, the method further comprises one or more purification steps (v) to ensure that solvents, by-products, impurities, or un-reacted products are removed from the composition. These may be conducted throughout any reaction steps and more than one purification technique may be applied during the preparation of the composition.

In a preferred embodiment, such purification steps may include washes in aqueous media. Phase separation during water washings can be improved by the use of salts solubilized in the aqueous phase (e.g. from about 50 to about 500 g/L salt aqueous solution, preferably about 300 g/L salt, for example sodium chloride, aqueous solution). According to a preferred embodiment, the water washing is salted water washing. Examples of salts include, but are not limited to, sodium chloride or potassium chloride.

Alternatively, purification steps may be carried out using acidic or basic aqueous solutions such as aqueous hydrogen chloride solution (from about 0.1N to 5N) or aqueous sodium carbonate solution (from about 10% w/w to saturated).

According to a preferred embodiment, such purification steps may be conducted either by solvent evaporation or supercritical carbon dioxide extraction.

Figures 1A and 1B illustrate a synthetic method according to an embodiment of the invention. The polymer backbone is prepared from polyethylene glycol and a diacid and the end groups are reacted with glycerol. Acrylation is achieved by reaction with 2-isocyanatoethylmethacrylate. Functionalization is achieved using 2-isocyanatoethyl-acrylate, followed by reaction with diethylamine and acidification with acetic acid.

In an embodiment, the method of the invention comprises the steps of:

i) polymerization of the diol and the diacid to provide the polymeric backbone;

ii) reaction of the polymeric backbone with an amine to provide a polymeric backbone functionalized with nitrogen-containing groups;

iii) reaction of the polymeric backbone with the polyol to provide additional hydroxy terminal groups;

iv) activation of the polymeric backbone to provide a pre-polymer comprising activated groups; and

v) acidification of the pre-polymer to obtain nitrogen-containing functionalized groups.

Figures 2A and 2B illustrate an alternative synthetic method according to an embodiment of the invention. The polymer backbone is prepared from polyethylene glycol and a diacid. The backbone is reacted with diethylethylenediamine (DEDA). The end groups are reacted with glycerol. Acrylation is achieved by reaction with 2-isocyanatoethylmethacrylate. Functionalization is achieved via acidification of the amine groups introduced via the diethylenediamine.

Uses

## Tissue fixation and sealing

The composition according to the invention may be used for joining or sealing targeted surfaces including tissue, graft material such as PTFE-based graft, medical devices, surgical patch or any combination thereof. The method for joining or sealing targeted surfaces comprises applying the composition to the surface and curing the composition. When used as sealant, the composition is able after curing to prevent leaking (e.g., of fluid or gas) by forming a barrier or filling a void volume.

Unlike conventional tissue adhesives that spontaneously activate during application or in the presence of water, or adhesives that are hydrophilic and thus are subject to washout prior to curing, the composition according to the invention can be applied to wet substrates without activation or displacement. The composition can also be applied to dry substrates.

Besides fixation and sealing of wet biological tissue, the composition may fix to and seal a variety of hydrophilic or hydrophobic substrates, natural or synthetic, including polyethylene terephthalate, expanded polyethylene terephthalate, polyester, polypropylene, silicones, polyurethanes, acrylics, fixed tissue (e.g., pericardium), ceramics or any combinations thereof.

The composition may also be used for joining tissue to the surface of a medical device or maintaining medical device, for example biocompatible implant (e.g. nerve conduit) in place *in vivo.* The composition can be used in medical devices, either as part or all of a device or to fix a device to tissue. The method for joining tissue to the surface of a medical device comprises applying the composition to the surface of the tissue and/or medical device and curing the composition. The composition can also be used to join tissue, including one or more tissue *in vivo.*

Surgical compositions comprising the composition according to the invention can also be used for other applications. Examples of applications include to stop bleeding, for example, due to a wound or trauma, during surgery such as after suturing a graft to a vessel, or after vascular access in endovascular procedures. The composition does not need to be removed before the surgeon sutures the wound closed since it will degrade over time. Other types of wounds that can be treated include, but are not limited to, wounds that leak, wounds that are hard to close or that fail to heal properly through normal physiologic mechanisms. The application can be performed both inside or outside the body, for human or veterinary use.

The composition according to the invention can also be fabricated into a biodegradable stent. The stent can increase the diameter of a blood vessel to increase flow through the vessel, but since the stent is biodegradable, the blood vessel can increase in diameter with a reduced risk of thrombosis or covering the stent with scar tissue, which can re-narrow the blood vessel. The composition can cover an outer surface of a stent to help fix the stent to a vessel wall in a manner that is less damaging to the tissue than an uncovered stent or avoid its displacement inside the body. Similarly, the composition can cover the surface of any devices which are in contact with tissue to provide a suitable interface that can join to tissue.

The composition according to the present invention can be used in a variety of other applications where fixing or sealing are required. These include, but are not limited to, air leaks following a lung resection; to reduce the time for surgical procedures; to seal dura; to ease laparoscopic procedures; as a degradable skin composition; as a hernia matrix to prevent or to reduce the need for stables or tacks; to prevent blood loss; to manipulate organs or tissues during surgical procedures; to secure corneal transplants in place; to patch a heart to deliver drugs and/or to reduce dilation of the heart after myocardial infarction; to attach another material to a tissue; to augment sutures or staples; to distribute forces across tissue; to prevent leaks; as a barrier membrane on the skin to prevent evaporation of water from burnt skin; as a patch for delivery of anti-scar or antimicrobial medication; to attach devices to tissue; to attach devices to mucus membrane as a tape to secure devices within an oral cavity, such as to hold dentures and oral appliances; as a tape to anchor soft tissue to bone; to prevent the formation of holes in tissue; and to enhance/augment mechanical properties of tissues, etc.

**Delivery of bioactive molecules**

The composition according to the invention described may also contain one or more pharmaceutical, therapeutic, prophylactic, and/or diagnostic agents that are released during the time period that the material functions as a sealant or fixing composition. The agent may be a small molecule agent, for example, having molecular weight less than 2000, 1500, 1000, 750, or 500 Daltons, a biomolecule, for example peptide, protein, enzyme, nucleic acid, polysaccharide, growth factors, cell adhesion sequences, such as RGD sequences or integrins, extracellular matrix components, or combinations thereof. Exemplary classes of small molecule agents include, but are not limited to, anti-inflammatories, analgesics, antimicrobial agents, and combinations thereof. Exemplary growth factors include, without limitation, TGF-$\beta$, acidic fibroblast growth factor, basic fibroblast growth factor, epidermal growth factor, IGF-I and II, vascular endothelial-derived growth factor, bone morphogenetic proteins, platelet-derived growth factor, heparin-binding growth factor, hematopoetic growth factor, peptide growth factor, or nucleic acids. Exemplary extracellular matrix components include, but are not limited to, collagen, fibronectin, laminin, elastin and combinations thereof. Proteoglycans and glycosaminoglycans can also be covalently or non-covalently associated with the composition of the present invention.

**Tissue support**

The composition according to the invention can be used to create tissue repair supports to serve a mechanical function within the body of patient in need thereof

The present invention further provides methods of use and use of the composition according to the present invention for preparing printing resin (e.g. 3D printing resin) and for producing shaped objects by a variety of techniques known in the art, including 3D printing. The invention further provides printed (e.g. 3D printed) shaped objects obtained by using the composition of the invention. Accordingly, the composition of the invention can be used for forming shaped articles such as for example biocompatible implant (e.g. nerve conduit). The shaped articles may be produced by a variety of fabrication techniques known in the art, including 3D printing. Such articles may exert functions, such as holding two tissues together or positioning the tissue in a specific position inside or outside the body. The shaped object may have micro or nanoscale resolution.

According to another embodiment, the composition of the invention is applied to any substrate having a surface, more particularly a tissue repair support, such as a surgical patch, for example a mesh substrate (e.g. hernia mesh substrate).

According to another embodiment, tissue can be coated with a layer of the composition of the invention, for example, the lumen of a tissue, such as a blood vessel to prevent restenosis, reclosure or vasospasm after vascular

intervention.

The composition may also contain one or more types of cells, such as connective tissue cells, organ cells, muscle cells, nerve cells, and combinations thereof. Optionally, the material is seeded with one or more of tenocytes, fibroblasts, ligament cells, endothelial cells, lung cells, epithelial cells, smooth muscle cells, cardiac muscle cells, skeletal muscle cells, islet cells, nerve cells, hepatocytes, kidney cells, bladder cells, urothelial cells, chondrocytes, and bone-forming cells. The combination of cells with the material may be used to support tissue repair and regeneration.

**Anti-adhesion barriers**

The composition according to the invention herein described can be applied to reduce or prevent the formation of adhesions after surgical procedures. For example, the composition can be applied to prevent adhesion of brain tissue to the skull after brain surgery or implantation of devices to prevent peritoneal adhesion.

**Other applications**

The compositions can also be used to coat tools, such as surgical instruments, for example, forceps or retractors, to enhance the ability of the tools to manipulate objects. The compositions can also be used in industrial applications where it is useful to have a degradable material that is biocompatible, for example, to reduce potential toxicity of the degradation products, such as marine applications, for example, in underwater use or attaching to the surface of boats.

The present invention will now be illustrated, but in no way limited, by reference to the following examples.

EXAMPLES

**Pre-polymer synthesis**

**Preparation of polymer from PEG200 and diacid**

Polyethylene glycol (PEG200, 1 eq.) and diacid (1 eq.) were placed in a round-bottom flask, and stirred in an oil bath pre-heated to different temperatures depending on the diacid (see **Erreur** ! **Source du renvoi introuvable.** below). After complete melting of the mixture, the flask was transferred to a pre-heated oil bath at 130°C and 0.25 or 0.50% eq. mol. of paratoluenesulfonic acid (pTSA) catalyst was added to the flask depending on the diacid (see **Erreur** ! **Source du renvoi introuvable**.). The flask was placed under dynamic vacuum, with vacuum set at 15 mBar. The reaction was carried out until the targeted number average molecular weight was reached. The [COOH] content was determined by $^1$H-NMR. Reaction temperature and catalyst content could be increased in order to accelerate the polymerization and accommodate working hours.

Polymers with Mn (number average molecular weight) ranging from 892 to 11667 g/mol were prepared. Viscosity was measured and ranged from below 550 to above 150 000 cP. [COOH] ranged from 0.05 to 1.04 mmol/g. Figure 3 shows the variation of $M_n$ with [COOH] content.

Table 1 - Reaction parameters for the reaction of PEG200 and diacid

| Diol | Diacid | Melting temperature prior reaction (°C) | Catalyst amount (% eq. mol.) |
|---|---|---|---|
| PEG200 | Sebacic acid | 130 | 0.25 |
| PEG200 | Pimelic acid | 160 | 0.50 |
| PEG200 | Adipic acid | 155 | 0.25 |
| PEG200 | Glutaric acid | 130 | 0.25 |
| PEG200 | Succinic acid | 185 | 0.25 |

**Reaction of pre-polymer with polyol by Steglich esterification**

The polymer was weighed in a flask. The polyol (4.0 eq/COOH) and N,N-dimethylpyridin-4-amine (DMAP, 0.5eq/COOH) were added with 5 mL/g of polymer of THF and the mixture was stirred at room temperature until a clear homogeneous mixture was obtained. N,N'-dicyclohexylcarbodiimide (DCC, 2.0eq/COOH in THF) was added and the mixture was stirred at room temperature. After twenty four hours, the solution was filtered and concentrated under reduced pressure. The residue was resuspended in DCM (4 mL/g of residue). The solution was washed once with 0.5N HCl in water in a separating funnel. The organic layer was collected and washed once with saturated $NaHCO_3$. The organic layer was

collected and washed once with distilled water. The cloudy yellow organic phase was collected, dried with $MgSO_4$ then filtered and concentrated under reduced pressure.

Polymers with Mn ranging from 1029 to 18168 g/mol were prepared. [OH] ranged from lower than 0.41 to above 2.25 mmol/g. Figure 4 shows the variation of $M_n$ with [OH] content.

The [OH] value was measured using $^{19}$F-NMR. The polymer was mixed with 4-fluorophenylisocyanates, leading to reaction with hydroxy groups within 10-15 minutes. An internal standard ($\alpha,\alpha,\alpha$-trifluorotoluene) was added before NMR analysis.

**Reaction of pre-polymer with polyol by polycondensation**

The polymer (1.0 eq.) and the polyol were placed in a round-bottom flask and stirred in a pre-heated oil bath (see **Erreur** ! **Source du renvoi introuvable.** below for reaction parameters). After complete melting of the mixture, the flask was placed under dynamic vacuum, with vacuum set at 15 mBar, and allowed to react. The reaction was carried out until the polyol peak was not visible anymore on GPC chromatogram. In the case of glycerol addition, the mixture was dissolved in EtOAc (1.3 mL/g of polymer) and washed twice with deionized water. The organic phase was collected, dried with $MgSO_4$ then filtered and concentrated under reduced pressure. In the case of TMTPE450 addition, no washes were performed.

Polymers with Mn ranging from 1184 to 8466 g/mol were prepared (TBE-328 = example 1, TBE-229 = example 2, TBE-238 = example 4, TBE-225 = example 10).

Table 2 - Reaction parameters for the reaction of pre-polymer with polyol by polycondensation

| Polymer | Polymer equivalent | Polyol | Polyol equivalent | Temperature (°C) |
|---|---|---|---|---|
| PEG200-SA | 1.0 eq. | Glycerol | 4.0 eq. | 130 |
| PEG200-SA | 1.0 eq. | TMTPE450 | 1.0 eq. | 180 |

**Reaction of hydroxy terminal groups with 2-isocyanatoethyl-methacrylate**

The polymer was weighed and mixed with 4-methoxyphenol (MEHQ, 1wt%/polymer) in ethyl acetate. The mixture was heated to 80°C and stirred until complete homogenization. 2-isocyanatoethyl-methacrylate (dependent on target for [A]) was added, and the mixture stirred for twenty four hours.

**Reaction of hydroxy terminal groups with 2-isocyanatoethyl-acrylate, diethylamine and acetic acid**

2-Isocyanatoethyl-acrylate (dependent on target for [$N^+$]) was added to the (meth)acrylated solution at 80°C. The mixture was stirred for twenty four hours. Then, the solution was cooled down to 55°C. DEA was added (4 eq./ 2-Isocyanatoethyl-acrylate) and the mixture stirred for twenty hours. The solution was cooled down to room temperature. Acetic acid (2 eq./DEA) was added to the mixture and stirred for 5 minutes. Then, the solution was washed once with 50% brine in water solution. The clear yellow organic phase was recovered, dried with $MgSO_4$ and filtered.

**Purification**

The solution was concentrated up to 50%wt. TPO (40000 ppm), MEHQ (4000 ppm) and blue dye (55 ppm) was loaded to the mixture. The solution was purified by supercritical $CO_2$ extraction (solvent ratio: 150, stirring: 50 RPM, flow: 20 g/min, temperature: 40°C). The pure polymer was defoamed at low pressure (15 mbar) and stored at -20°C.

**Polymer properties**

Pre-polymers were made according to the methods described above, using the diols, diacids and polyols outlined in corresponding tables. The amount of acrylate groups [A] and the amount of functionalized groups [N+] were measured using 1H NMR. The average molecular weight was measured using Gel Permeation Chromatography equipped with a refractive index and polystyrene calibration standards. TMTPE 450 is trimethylolpropane ethoxylate with average molecular weight of 450 g/mol.

**Case 1 (Figures 5A, 5B, 5C and 5D)**

The mechanical properties of the cured polymers (Young's modulus (YM), tensile strain, tensile strength and

toughness) were measured by uniaxial tensile testing. Cured polymer films with 0.4 mm thickness were prepared by putting approximately 0.8 g of pre-polymer on a glass slide with two 0.4 mm metal spacers (Mitutoyo). A second glass slide was placed on top and the two glass plates were set with clamps on the metal spacers. A sample was put in a blue light curing chamber (405 nm) and exposed for 30 seconds. The cured polymer film was recovered, and specimens were cut from the cured polymer film using a puncher.

Specimens were clamped between pneumatic grips (Low-force grips 2712-051, Instron, Norwood, Mass.) loaded on 10N load cell (Series S-beam Static Load Cell 2519-10N, Instron, Norwood, Mass.) attached to a vertical mechanical testing machine (3340 Series Single Column, Instron, Norwood, Mass.). A uniaxial tensile test was performed at the rate of 5 mm per min, with specimen break the test stop criterion.

The load vs extension curve, the stress vs strain curve as well as the load at break were recorded. The YM (MPa) was the slope of the linear regression performed on the linear elastic region of the stress vs strain curve. The tensile strain (%) was the strain recorded at specimen's break. The tensile strength (MPa) was the load recorded at specimen's break. The toughness (AUC, Area Under the Curve) was calculated as the area under the stress *vs* strain curve. The results presented are the mean of six replicates for each cured polymer. The comparative polymer was prepared according to the methods disclosed in WO 2021/078962.

Results are presented in Figures 5A, 5B, 5C and 5D. Examples 1-3 demonstrate the wide range of mechanical properties achievable using the described method by selecting appropriate monomers, molecular weight Mn, concentration of acrylate groups [A], and amount of functionalized groups [N+]. Example 1 exhibits a Young's Modulus approximately 30 times greater than that of the comparative polymer. Example 2 demonstrates a strain approximately 25 times higher than the comparative polymer. Lastly, Example 3 displays a Young's Modulus almost 170 times lower than the comparative polymer. Figures 5A, 5B, 5C and 5D illustrate the standard stress/strain curve obtained through the assessment of mechanical properties with each example of polymers according to the invention positioned relative to the comparative polymer.

<u>**Case 2 (Figure 6)**</u>

This case study demonstrates the modularity of the method. By modulating the nature of diol, diacid, and polyol, as well as the concentration of activated groups [A], functionalized groups, and molecular weight, specific mechanical properties can be attained. This feature enables the modification of secondary performance while targeting specific mechanical properties.

Examples 4-5 showcase similar mechanical properties despite differences in polyol nature, concentration of activated groups [A], and molecular weight Mn.

Examples 6-7 exhibit equivalent mechanical properties while varying in the concentration of activated groups [A], concentration of functionalized groups [N+], and molecular weight.

Examples 8-9 demonstrate equivalent mechanical properties with variations in polyol nature, concentration of activated groups [A], and molecular weight.

Examples 10-11 display equivalent mechanical properties while differing in polyol nature, concentration of activated groups [A], and molecular weight.

<u>**Case 3 (Figures 7A and 7B)**</u>

Accelerated degradation experiments were performed by preparing cured polymer films with 0.8 mm thickness by putting approximately 0.8 g of pre-polymer on a glass slide with two 0.8 mm metal spacers (Mitutoyo). A second glass slide was placed on top and the two glass plates were set with clamps on the metal spacers. A sample was put in a blue light curing chamber (405 nm) and exposed for 30 seconds at maximum intensity. The cured polymer film was carefully recovered, and disks of 6 mm diameter were cut from the cured polymer film using a biopsy puncher. The disks were incubated at 97°C in 2 mL Eppendorf tubes in 2 mL of PBS in dry block heaters. The pH was checked daily, and the PBS was renewed if pH was measured below 7.5. The accelerated degradation was followed for 7 days. At each timepoint (1 day, 3 days and 7 days), 4 disks were taken out of the media and placed to dry for 24h at 60°C in an oven. Dry mass (%) was calculated as follows:

$$dry\ mass\ \% = \frac{m_{dry(t)}}{m_{t0}} \times 100 \quad .$$

Where $m_{t0}$ is the dry mass (mg) of sample at t0 and $m_{dry(t)}$ is the dry mass (mg) of degraded sample at timepoint t

This case study provides a more comprehensive example of modulating secondary performance, specifically degradation, by adjusting one of the structural factors such as monomers, [A], [N+], and/or Mn.

Examples 15-19 demonstrate that by utilizing different diacids, accelerated degradation at 97°C can be controlled while maintaining equivalent mechanical properties. In particular polymers synthetized with succinic acid (Example 18), glutaric acid (Example 16) or adipic acid (Example 19) present accelerated degradation. This might be advantageous in some uses.

The parameters [A], [N+], and Mn are also held constant across these examples.

## Case 4 (Figures 8A and 8B)

Examples were tested for fixation strength measurement according to the following pull off protocol. Pull off mold fixation strength testing (at 90°C) was performed on a mechanical tester with fresh porcine epicardial tissue. The tissue was kept in PBS to assure that it remained wet during testing. Silicon sheet of 0.4 mm thickness with a 3 mm diameter hole was used as mold to deposit a disk of pre-polymer of controlled dimension (3 mm diameter and 0.4 mm thickness) on the porcine epicardial tissue. Curing of the pre-polymer disk was performed in a blue light (405 nm) chamber for 30s maximum. Borosilicate pin was glued to the cured polymer disk to serve as a fixation point for the upper grip of the mechanical tester. The pull off procedure involved grip separation at a rate of 8 mm/min, causing uniform cured polymer disk detachment from the tissue surface. Fixation strength was recorded as the maximum force observed before fixation failure, when a sharp decrease in the measured stress was observed.

This case study demonstrates that the desired fixation strength (with an average above 6 $N/cm^2$) can be achieved by independently adjusting the [N+] parameter, regardless of the selected monomers. In this particular case, [A] and Mn are considered equivalent. However, similar trends are observed in examples with varying [A] and Mn, not shown here.

## Case 5 (Figures 9A and 9B)

This case shows that improved fixation strength can be reached independently of the functionalization method.

## Case 6 (Figure 10)

For assessing mechanical properties after incubation in a liquid, the cured polymer films with 0.4 mm thickness were immersed in a 150 mL amber bottle, in 100 ml of PBS, and placed on the static plate of an incubator for 24 h at 37°C. After 24 h, the samples were tested for mechanical properties on a mechanical tester.

This case 6 illustrates that by appropriately selecting the [N+] parameter, mechanical properties, particularly tensile strain, can be preserved after incubation in PBS for 24 hours at 37°C, while achieving the desired fixation strength.

Example 20, which has no [N+], exhibits a fixation strength of 0 and its tensile strain remains unaffected by incubation in PBS.

Example 21, with a [N+] of 0.12 mmol/g demonstrates an enhanced fixation strength, while still showing no effect on tensile strain.

Examples 22-23, with [N+] values of 0.16 and 0.20 mmol/g respectively, achieve the improved fixation strength, and their tensile strain is also unaffected by incubation in PBS.

Example 24, with a [N+] of 0.33 mmol/g, achieves good fixation strength; however, its tensile strain is affected by incubation, with a value twice as low as t0.

In all these batches, other metrics such as monomers, polyol, [A], and Mn are considered equivalent.

## Case 7 (Figure 11)

Resistance to bending was evaluated with a cyclic method. A polymer conduit was created with the use of a mold around a linen rope. This conduit was placed and secured on a bending device and subjected to incremental cycles of bending until failure/breaking of the polymer conduit with a mechanical tester. The test consists of 4 displacement phases of 4, 4.5 ,5.5 and 6 mm of 10 cycles each. Increasing the intensity of the bend at each phase at 20mm/min for 40 cycles.

Four replicates per Examples were performed and the average number of cycles at which the sample cohesively breaks was measured.

This case shows that example 35-38 can withstand the 40 cycles of the bending tests while comparative polymer fails at an average of 10 cycles.

## Claims

1. A pre-polymer having a polymeric backbone derived from a diol and a diacid, wherein the pre-polymer comprises terminal groups derived from a polyol,

and wherein the pre-polymer comprises activated groups, and optionally further comprises functionalized groups, wherein the functionalized groups include a positively charged heteroatom.

2. The pre-polymer according to claim 1, wherein the diol is polyethylene glycol.

3. The pre-polymer according to any preceding claim, wherein the diacid is selected from succinic acid, glutaric acid, adipic acid, pimelic acid, suberic acid, azelaic acid, and sebacic acid, wherein each diacid may be substituted or unsubstituted.

4. The pre-polymer according to claim 3, wherein the diacid is substituted or unsubstituted sebacic acid.

5. The pre-polymer according to claim 1, wherein the polymeric backbone is derived from polyethylene glycol and sebacic acid.

6. The pre-polymer according to claim 1, wherein the polymeric backbone is of the general formula (I):

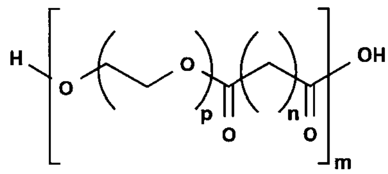

(I)

wherein n, p and m each independently represent an integer greater than 1.

7. The pre-polymer according to any preceding claim, wherein the polyol has 3, 4, 5 or 6 hydroxy groups.

8. The pre-polymer according to claim 7, wherein the polyol is selected from glycerol, trimethylolpropane ethoxylate, di(trimethylolpropane) and xylitol, and preferably is glycerol.

9. The pre-polymer according to any preceding claim, wherein the polymeric backbone is derived from polyethylene glycol and sebacic acid, and the terminal groups are derived from glycerol.

10. The pre-polymer according to any preceding claim, wherein the polymeric backbone is of the general formula (II):

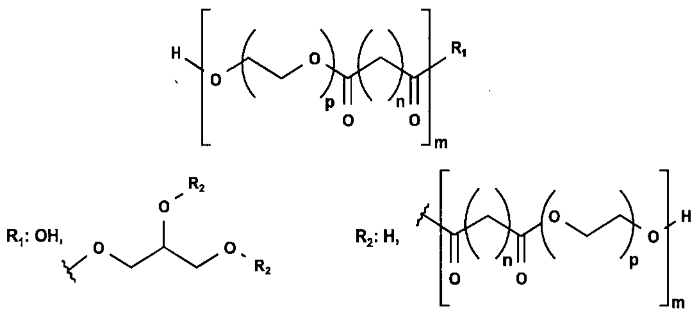

(II)

wherein n, p and m each independently represent an integer greater than 1.

11. The pre-polymer according to any preceding claim, wherein the activated groups are acrylate and/or methacrylate groups.

**12.** The pre-polymer according to any preceding claim, wherein the amount of activation is between 0.03 mmol/g and 4 mmol/g.

**13.** The pre-polymer according to any preceding claim, wherein the amount of functionalized groups is between 0 mmol/g and 4 mmol/g, preferably between 0.2 mmol/g and 1 mmol/g.

**14.** The pre-polymer according to any preceding claim, wherein the pre-polymer comprises functionalized groups, wherein the functionalized groups include a positively charged nitrogen atom.

**15.** A composition comprising a pre-polymer according to any preceding claim.

**16.** The composition according to claim 15 further comprising an initiator.

**17.** A method for preparing a pre-polymer according to any one of claims 1 to 14, comprising steps of:

i) polymerization of the diol and the diacid to provide the polymeric backbone;
ii) reaction of the polymeric backbone with the polyol to provide additional hydroxy terminal groups;
iii) activation to provide activated groups; and
iv) optionally, functionalization with functionalized groups, wherein the functionalized groups include a positively charged heteroatom.

**18.** A method according to claim 17, wherein the activation in step iii) is achieved by acrylation of terminal hydroxy groups, preferably using an isocyanate acrylate compound.

**19.** A method according to claim 17 or claim 18, wherein the functionalization in step iv) is achieved by a sequence of acrylation, amination and acidification.

**20.** A method for preparing a pre-polymer according to any one of claims 1 to 14, comprising steps of:

i) polymerization of the diol and the diacid to provide the polymeric backbone;
ii) reaction of the polymeric backbone with an amine to provide a polymeric backbone functionalized with nitrogen-containing groups;
iii) reaction of the polymeric backbone with the polyol to provide additional hydroxy terminal groups;
iv) activation of the polymeric backbone to provide a pre-polymer comprising activated groups; and
v) acidification of the pre-polymer to obtain nitrogen-containing functionalized groups.

**21.** A method of curing a composition according to any claim 15 or 16, comprising a step of curing the composition with as stimulus, preferably with light in the presence of a photo-initiator.

**22.** A composition according to any claim 15 or 16 or a composition obtainable by a method according to claim 21, for use in a method of adhering or sealing tissue, or for adhering tissue to the surface of a medical device.

**23.** A cured composition obtainable by the method of claim 21.

**24.** Use of a composition according to claim 15 or claim 16 or a composition obtainable by a method according to any one of claims 21, in a method of fixing to or sealing tissue, or for fixing a medical device to the surface of a tissue.

**25.** Use of a composition according to claim 15 or claim 16 or a composition obtainable by a method according to any one of claims 21, for preparing printing resin or for producing shaped objects by 3D printing.

**26.** Printed shaped objects prepared using a composition according to claim 15 or claim 16, or a composition obtainable by a method according to claim 21.

**Figure 1A**

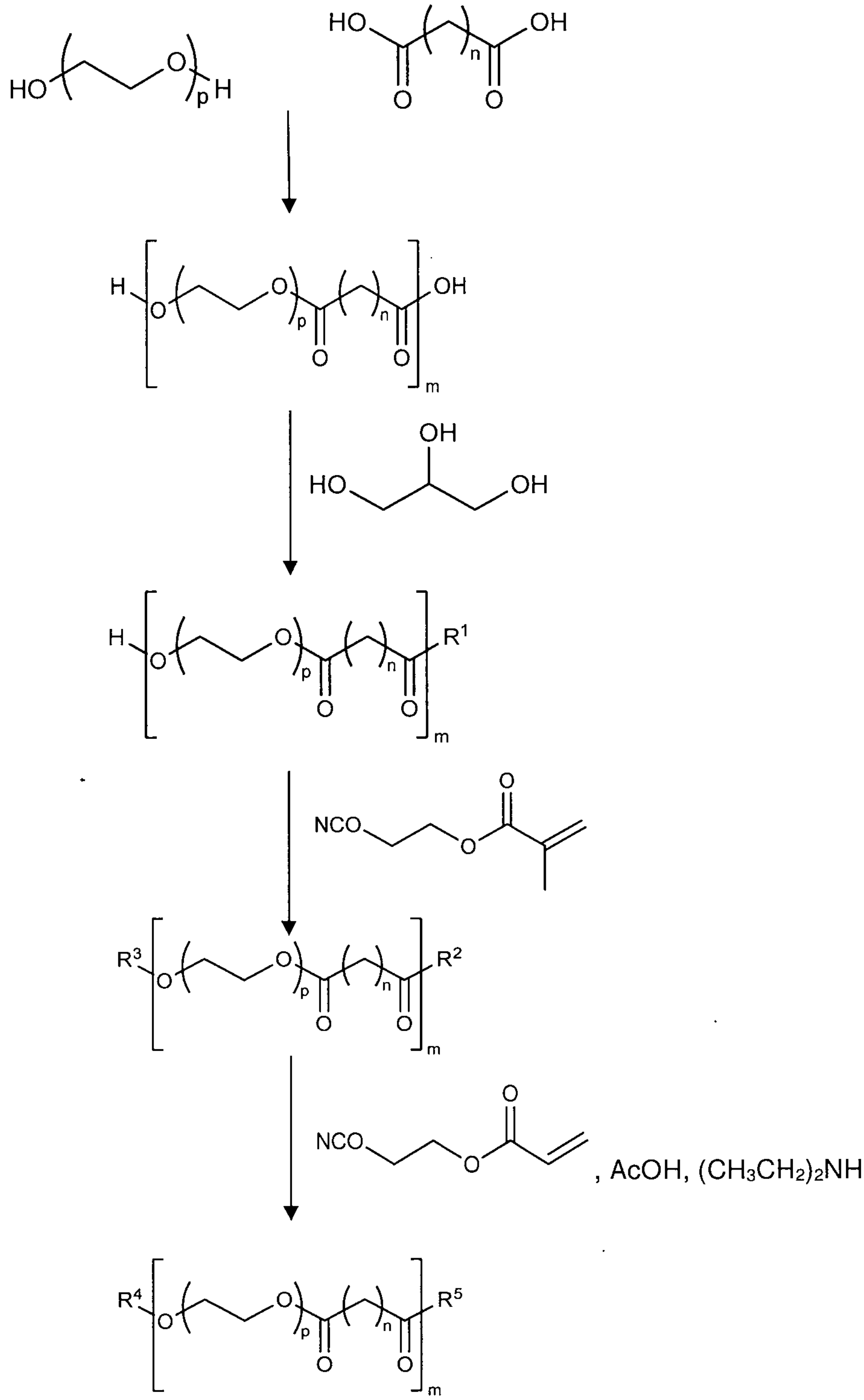

**Figure 1B**

$R^1$ = OH, 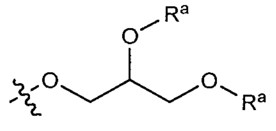

$R^a$ = H, 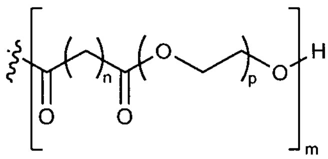

$R^2$ = OH, 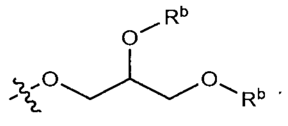

$R^3$ = H, 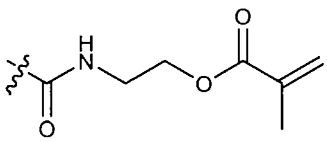

$R^b$ = H, 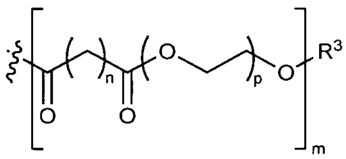 , 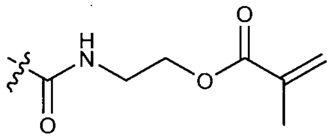

$R^4$ = H, 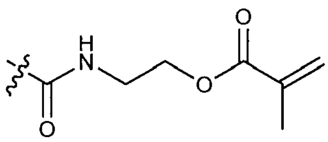 , 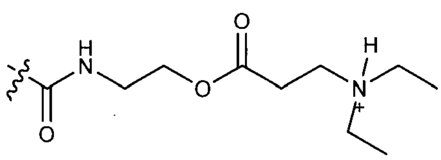

$R^5$ = OH, 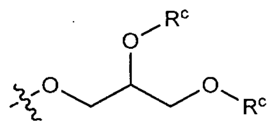

$R^c$ = H, 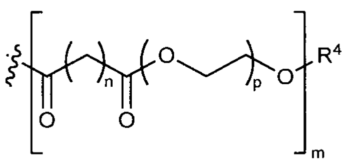 , 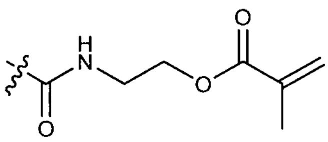

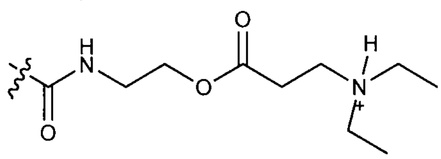

**Figure 2A**

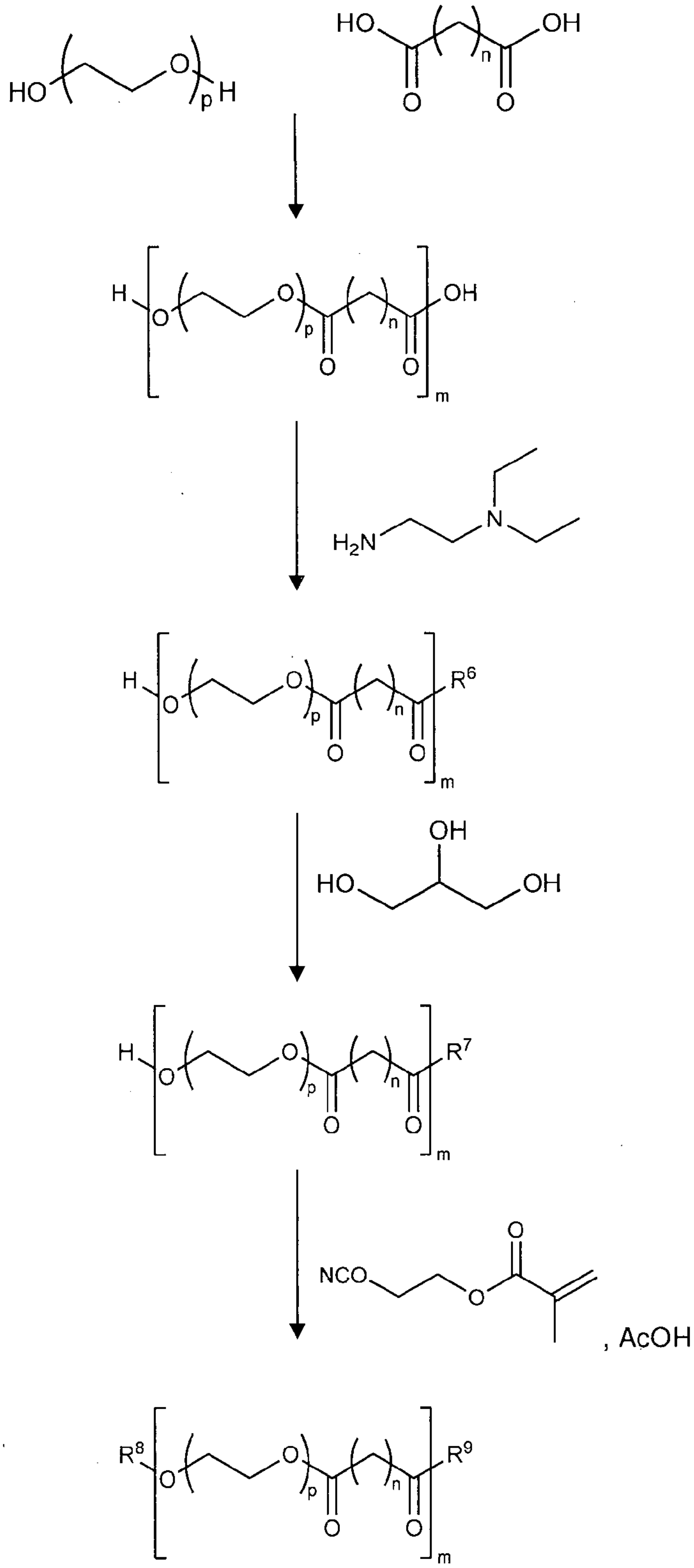

**Figure 2B**

$R^6$ = OH, 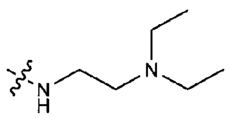

$R^7$ = OH, 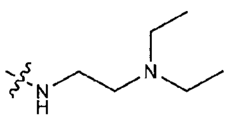, 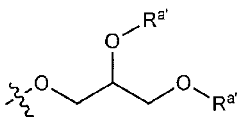

$R^{a'}$ = H, 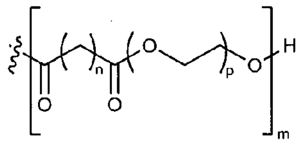

$R^8$ = OH, 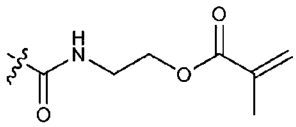

$R^9$ = OH, 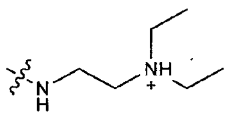, 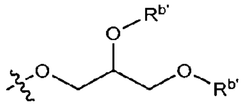

$R^{b'}$ = H, 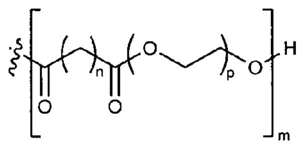, 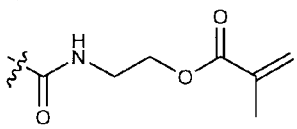

**Figure 3**

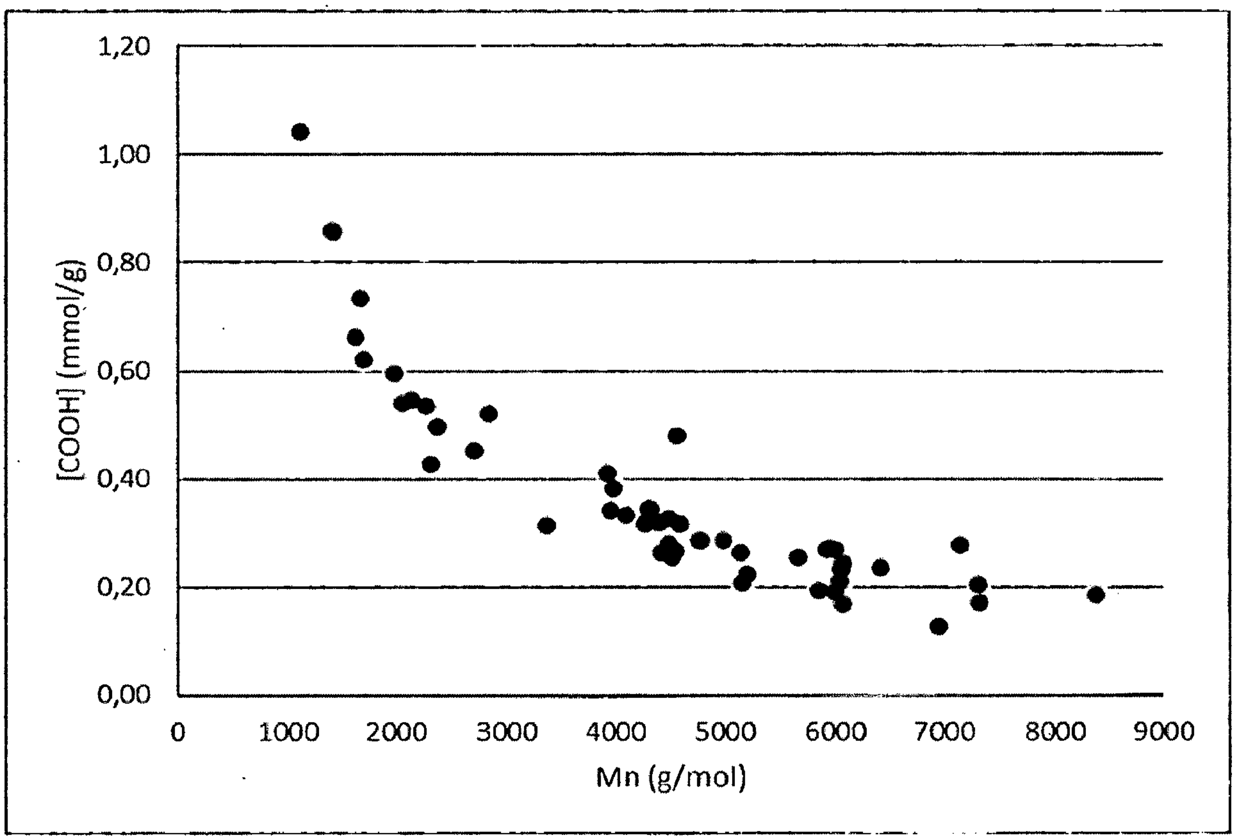
1,20
1,00
0,80
0,60
0,40
0,20
0,00
[COOH] (mmol/g)
0
1000
2000
3000
4000
5000
6000
7000
8000
9000
Mn (g/mol)

**Figure 4**

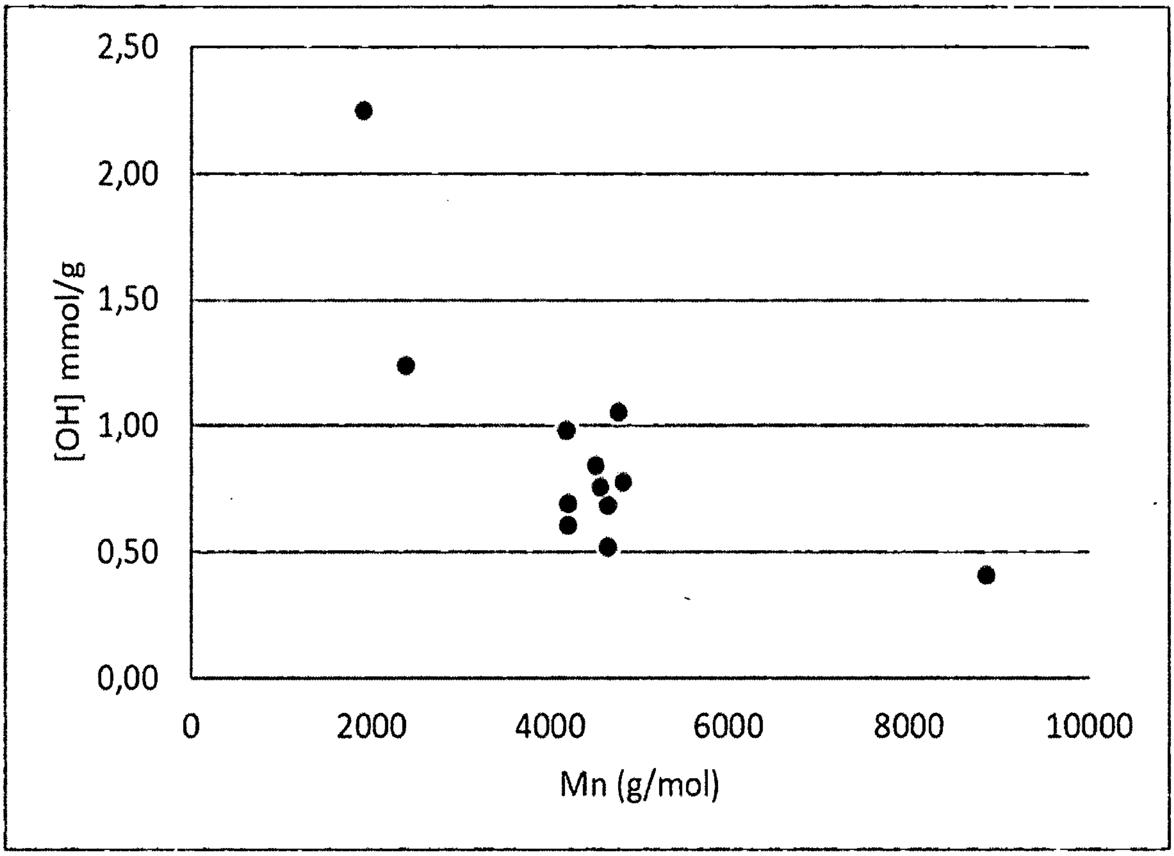
2,50
2,00
1,50
1,00
0,50
0,00
[OH] mmol/g
0
2000
4000
6000
8000
10000
Mn (g/mol)

Figure 5A

| Example # | Diol | Diacid | Polyol | [A] (mmol/g) | [N+] (mmol/g ) | Mn (g/mol) | Young's Modulus (MPa) | Tensile strain (%) | Tensile Strengt h (%) | Tough ness (AUC) |
|---|---|---|---|---|---|---|---|---|---|---|
| **Comparative polymer** | - | Sebacic acid | Glycerol | ~1 | ~0.7 | 1400-1700 | 5-6 | 10-20 | 0.70-1.99 | 0.06-0.08 |
| **Example 1** | PEG 200 | Sebacic acid | Glycerol | 3,61 | 0,00 | 1884 | 143,46 ± 9,13 | 20,4 ± 4.8 | 13,48 ± 2.11 | 1,78 |
| **Example 2** | PEG 200 | Sebacic acid | TMTPE 450 | 0,10 | 0,00 | 8953 | 0,14 ± 0.02 | 360,5 ± 32.5 | 0,24 ± 0.02 | 0,47 |
| **Example 3** | PEG 200 | Sebacic acid | Glycerol | 0,17 | 0,46 | 5536 | 0,03 ± 0.01 | 122,2 ± 45.0 | 0,05 ± 0.02 | 0,04 |

**Figure 5B**

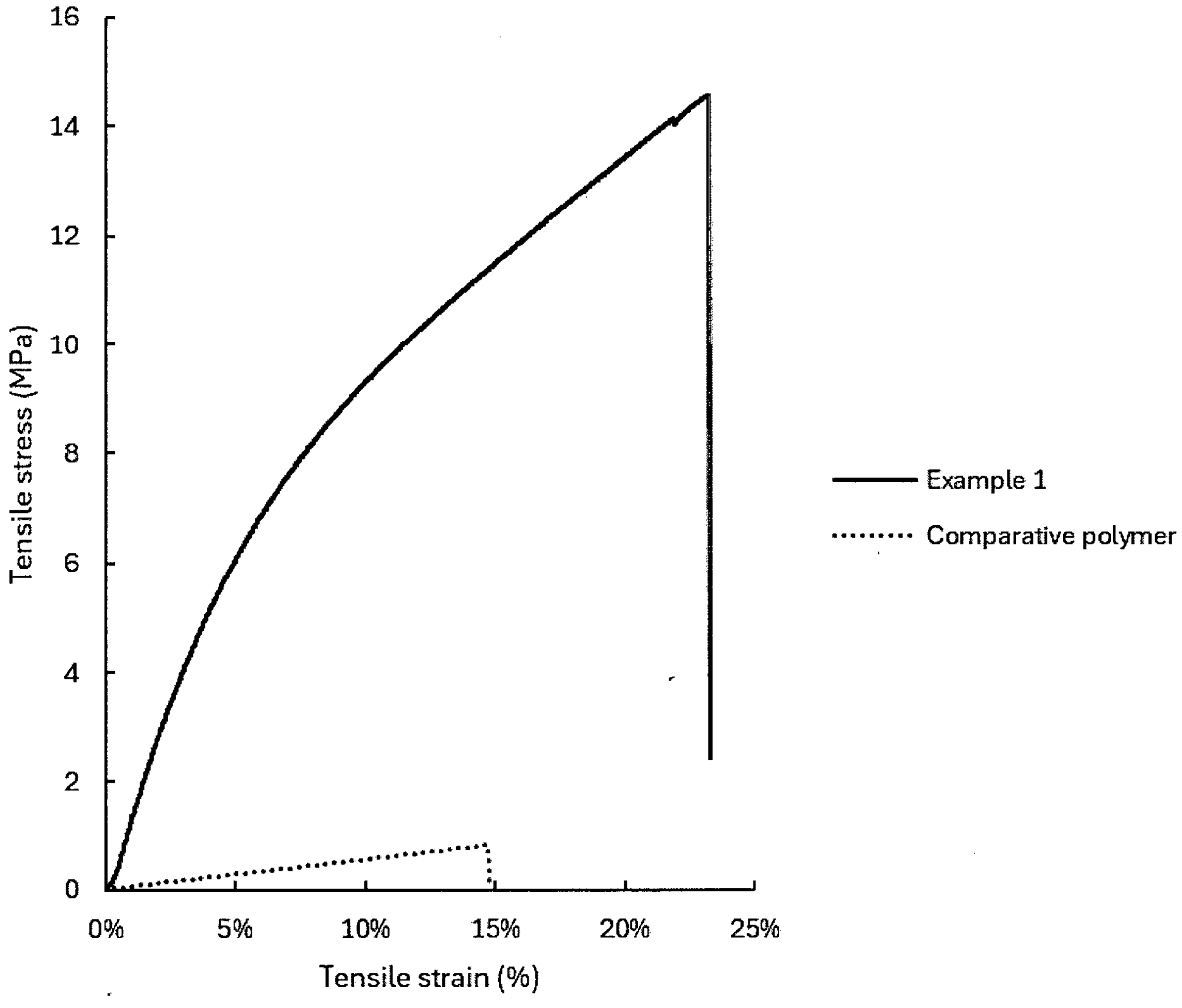
16
14
12
10
8
6
4
2
0
Tensile stress (MPa)
0%
5%
10%
15%
20%
25%
Tensile strain (%)
Example 1
Comparative polymer

**Figure 5C**

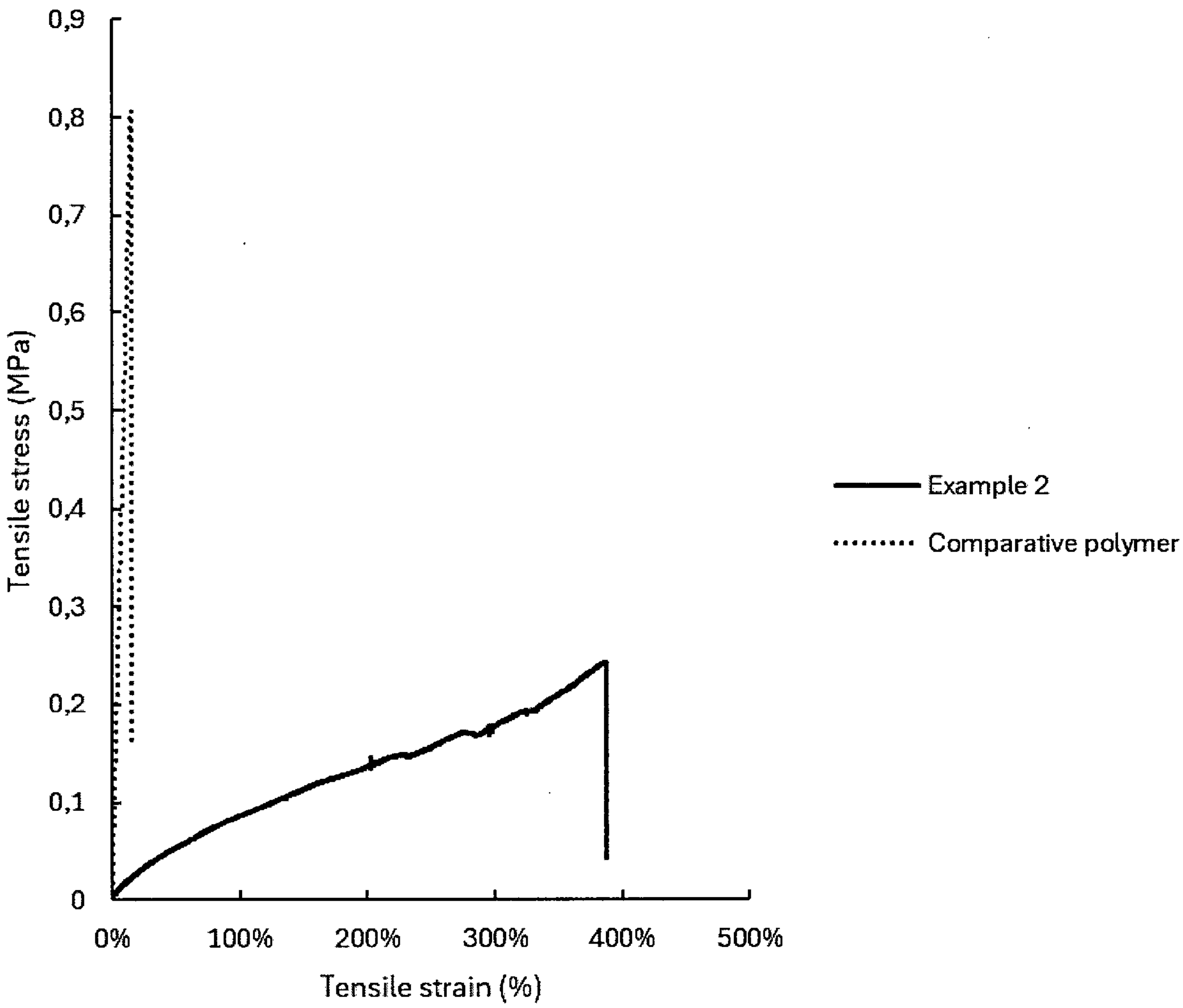
0,9
0,8
0,7
0,6
0,5
0,4
0,3
0,2
0,1
0
Tensile stress (MPa)
0%
100%
200%
300%
400%
500%
Tensile strain (%)
Example 2
Comparative polymer

**Figure 5D**

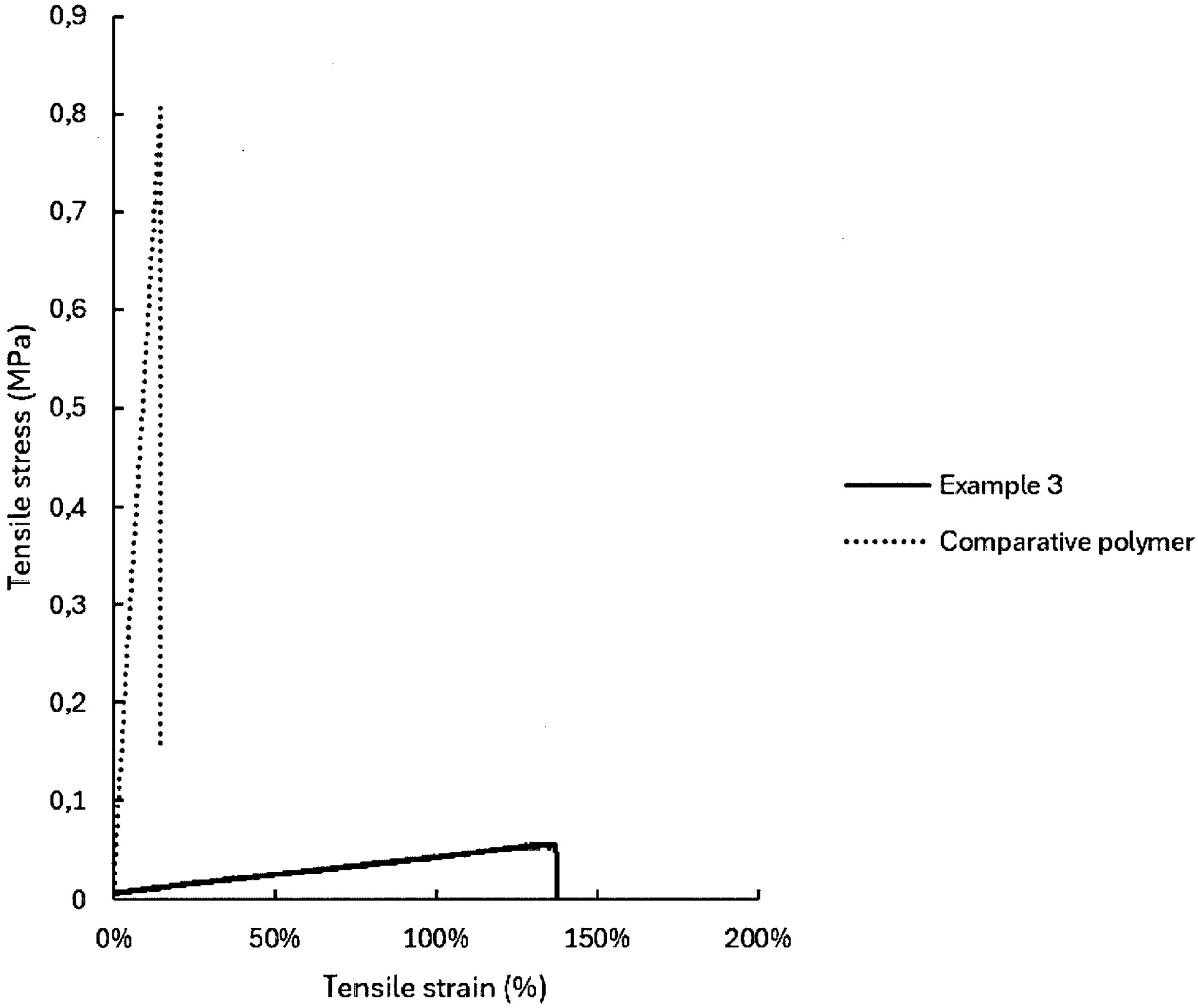
0,9
0,8
0,7
0,6
0,5
0,4
0,3
0,2
0,1
0
Tensile stress (MPa)
0%
50%
100%
150%
200%
Tensile strain (%)
Example 3
Comparative polymer

Figure 6

| Example # | Diol | Diacid | Polyol | [A] (mmol/g) | [N+] (mmol/g) | Mn (g/mol) | Young Modulus (MPa) | Tensile strain (%) | Tensile strength (MPa) | Toughness (AUC) |
|---|---|---|---|---|---|---|---|---|---|---|
| **Example 4** | PEG 200 | Sebacic acid | TMTPE 450 | 0,16 | 0,14 | 8785 | 0,86 ± 0.02 | 112,8 ± 25.3 | 0,50 ± 0.08 | 0,35 |
| **Example 5** | PEG 200 | Sebacic acid | Glycerol | 0,37 | 0,15 | 4643 | 0,95 ± 0.05 | 97,2 ± 26.8 | 0,76 ± 0.26 | 0,39 |
| | | | | | | | | | | |
| **Example 6** | PEG 200 | Sebacic acid | Glycerol | 0,32 | 0,00 | 9151 | 1,04 ± 0.02 | 141,4 ± 48.7 | 0,81 ± 0.21 | 0,72 |
| **Example 7** | PEG 200 | Sebacic acid | Glycerol | 0,27 | 0,39 | 6913 | 0,96 ± 0.06 | 148,1 ± 26.9 | 1,00 ± 0.27 | 0,75 |
| | | | | | | | | | | |
| **Example 8** | PEG 200 | Sebacic acid | Di(trimethy-lolpropane) | 0,40 | 0,00 | 5022 | 1,07 ± 0.03 | 150,1 ± 21.5 | 2,14 ± 0.64 | 1,19 |
| **Example 9** | PEG 200 | Sebacic acid | Glycerol | 0,22 | 0,00 | 10399 | 1,05 ± 0.02 | 202,7 ± 38.4 | 0,89 ± 0.16 | 1,06 |
| | | | | | | | | | | |
| **Example 10** | PEG 200 | Sebacic acid | TMTPE 450 | 0,17 | 0,00 | 9628 | 1,15 ± 0.08 | 79,9 ± 24.2 | 0,52 ± 0.09 | 0,26 |
| **Example 11** | PEG 200 | Sebacic acid | Xylitol | 0,52 | 0,00 | 5744 | 1,24 ± 0.02 | 74,6 ± 14.7 | 0,64 ±0.13 | 0,26 |
| | | | | | | | | | | |
| **Example 12** | PEG 200 | Sebacic acid | TMTPE 450 | 0.24 | 0.00 | 6624 | 1.01 ± 0.02 | 112.9 ± 33.1 | 0.74 ± 0.22 | 0.48 |
| **Example 13** | PEG 400 | Sebacic acid | TMTPE 170 | 0.37 | 0.00 | 4820 | 1.12 ± 0.01 | 71.7 ± 26.8 | 0.56 ± 0.19 | 0.24 |
| **Example 14** | PEG 600 | Sebacic acid | Glycerol | 0.41 | 0.00 | 4892 | 1.51 ± 0.07 | 65.5 ± 26.9 | 0.73 ± 0.28 | 0.27 |

Figure 7A

| Example # | Diol | Diacid | Polyol | [A] (mmol/g) | [N+] (mmol/g) | Mn (g/mol) | Young's Modulus (MPa) | Tensile strain (%) |
|---|---|---|---|---|---|---|---|---|
| **Example 15** | PEG 200 | Sebacic acid | Glycerol | 0.37 | 0.16 | 4295 | 0.89 ± 0.07 | 69.9 ± 13.9 |
| **Example 16** | PEG 200 | Glutaric acid | Glycerol | 0.31 | 0.17 | 4804 | 1.15 ± 0.05 | 107.9 ± 28.1 |
| **Example 17** | PEG 200 | Pimelic acid | Glycerol | 0.32 | 0.26 | 4727 | 1.03 ± 0.04 | 71.1 ± 18.1 |
| **Example 18** | PEG 200 | Succinic acid | Glycerol | 0.28 | 0.12 | 5119 | 1.15 ± 0.02 | 72.5 ± 24.9 |
| **Example 19** | PEG 200 | Adipic acid | Glycerol | 0.26 | 0.17 | 5090 | 0.58 ± 0.03 | 71.4 ± 11.6 |

**Figure 7B**

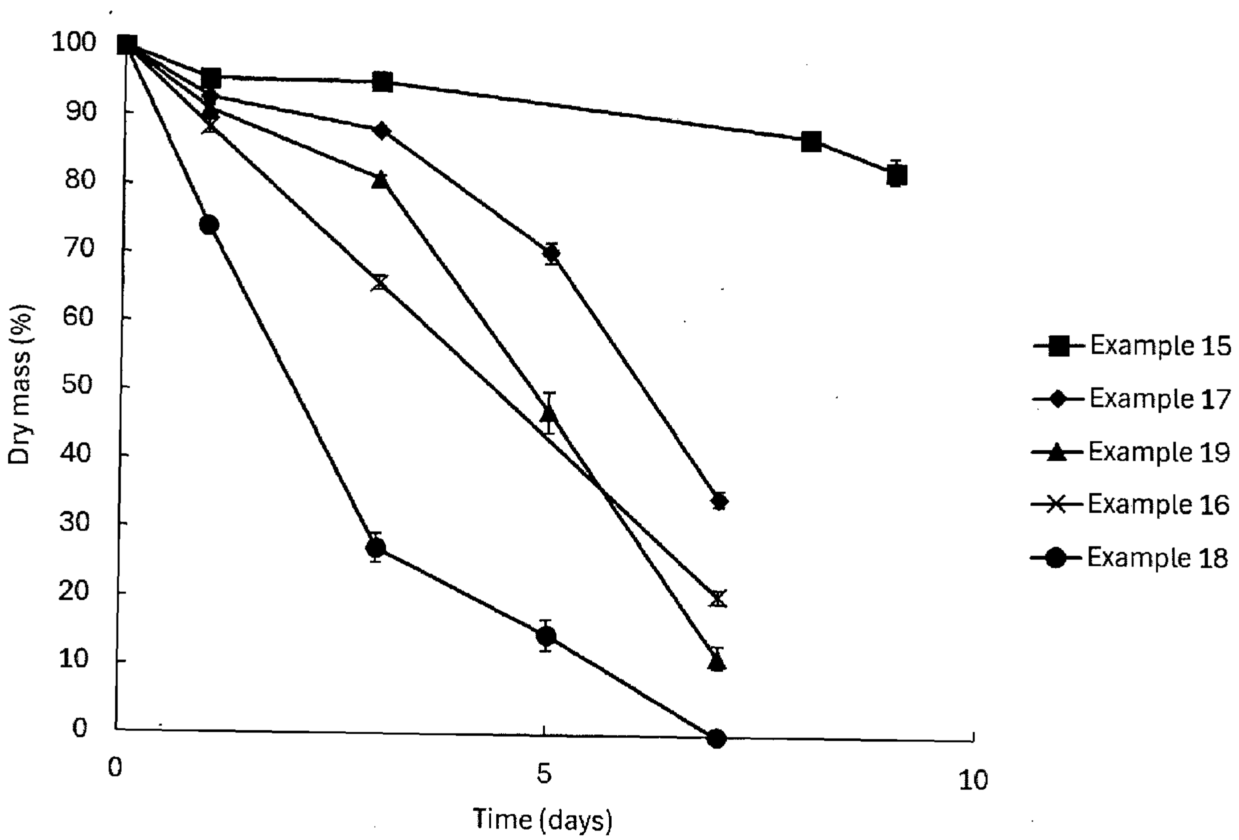
100
90
80
70
60
50
40
30
20
10
0
Dry mass (%)
0
5
10
Time (days)
Example 15
Example 17
Example 19
Example 16
Example 18

**Figure 8A**

| Example # | Diol | Diacid | Polyol | [A] (mmol/g) | [N+] (mmol/g) | Mn (g/mol) | Fixation strength (N/cm²) |
|---|---|---|---|---|---|---|---|
| **Example 20** | PEG 200 | Sebacic acid | Glycerol | 0,39 | 0,20 | 4128 | 8.9 ± 2.0 |
| **Example 21** | PEG 200 | Sebacic acid | Glycerol | 0,30 | 0,00 | 4838 | 0 ± 0 |
| **Example 22** | PEG 200 | Sebacic acid | Glycerol | 0,29 | 0,33 | 4709 | 5.9 ± 2.0 |
| **Example 15** | PEG 200 | Sebacic acid | Glycerol | 0,37 | 0,16 | 4295 | 8.1 ± 1.0 |
| **Example 23** | PEG 200 | Sebacic acid | Glycerol | 0,34 | 0,12 | 4803 | 1.4 ± 0.4 |
| **Example 24** | PEG 200 | Sebacic acid | Glycerol | 0,39 | 0,18 | 5065 | 9.5 ± 2.0 |
| **Example 25** | PEG 200 | Sebacic acid | Glycerol | 0,37 | 0,15 | 4643 | 9.8 ± 2.0 |
| **Example 18** | PEG 200 | Succinic acid | Glycerol | 0,28 | 0,12 | 5119 | 4.7 ± 3.2 |
| **Example 26** | PEG 200 | Succinic acid | Glycerol | 0,37 | 0,04 | 5476 | 0 ± 0 |
| **Example 27** | PEG 200 | Succinic acid | Glycerol | 0,30 | 0,11 | 4705 | 2.0 ± 0.9 |
| **Example 28** | PEG 200 | Succinic acid | Glycerol | 0,37 | 0,23 | 4365 | 6.6 ± 2.6 |
| **Example 29** | PEG 200 | Pimelic acid | Glycerol | 0,30 | 0,11 | 5035 | 1.5 ± 2.1 |
| **Example 30** | PEG 200 | Pimelic acid | Glycerol | 0,34 | 0,19 | 4232 | 6.4 ± 3.1 |
| **Example 17** | PEG 200 | Pimelic acid | Glycerol | 0,32 | 0,26 | 4727 | 2.5 ± 2.0 |

Figure 8B

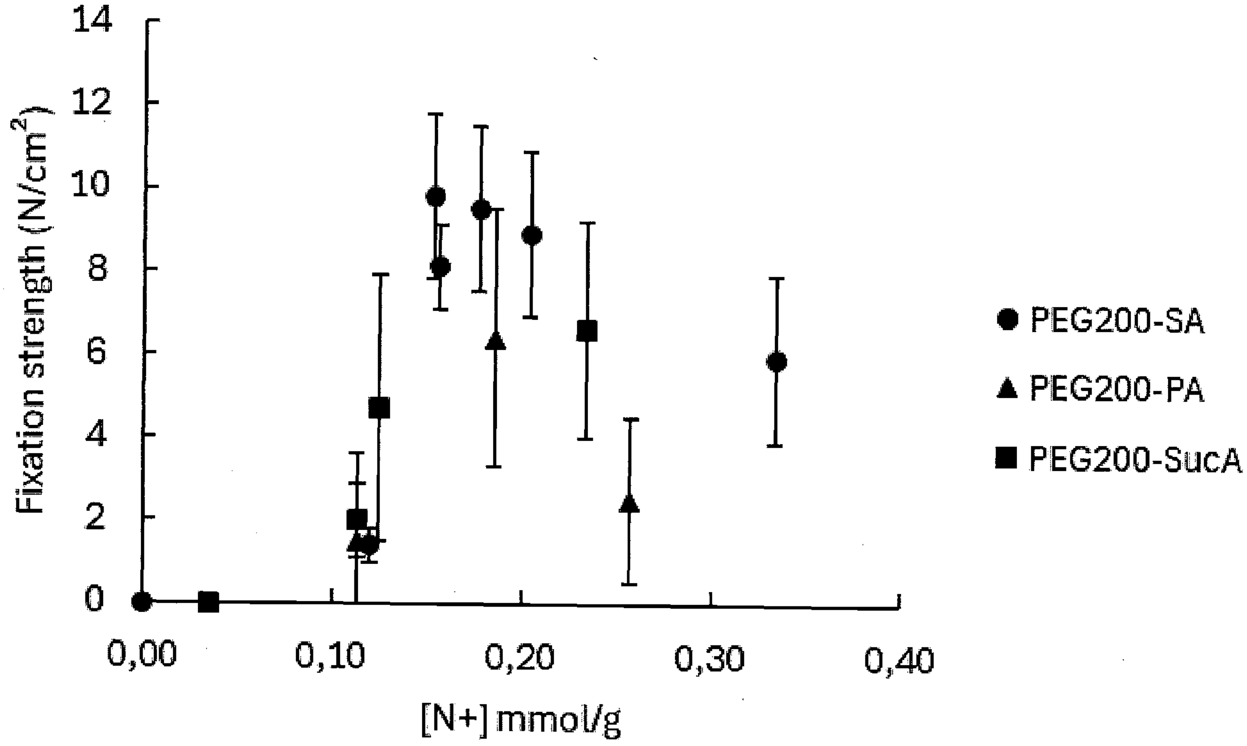
Fixation strength (N/cm$^2$)
14
12
10
8
6
4
2
0
0,00
0,10
0,20
0,30
0,40
[N+] mmol/g
PEG200-SA
PEG200-PA
PEG200-SucA

Figure 9A

| Example # | Diol | Diacid | Polyol | [A] (mmol/g) | Functionalization method | [N+] (mmol/g) | Mn (g/mol) | Fixation strength (N/cm²) |
|---|---|---|---|---|---|---|---|---|
| **Example 29** | PEG 200 | Pimelic acid | Glycerol | 0,30 | IsAc+DEA | 0,11 | 5035 | 1.5 ± 2.1 |
| **Example 30** | PEG 200 | Pimelic acid | Glycerol | 0,34 | IsAc+DEA | 0,19 | 4232 | 6.4 ± 3.1 |
| **Example 17** | PEG 200 | Pimelic acid | Glycerol | 0,32 | IsAc+DEA | 0,26 | 4727 | 2.5 ± 2.0 |
| **Example 31** | PEG 200 | Pimelic acid | Glycerol | 0,42 | DEDA | 0,15 | 4116 | 6.2 ± 5.1 |
| **Example 32** | PEG 200 | Pimelic acid | Glycerol | 0,51 | DEDA | 0,14 | 4506 | 7.2 ± 3.2 |
| **Example 33** | PEG 200 | Pimelic acid | Glycerol | 0,39 | DEDA | 0,18 | 4673 | 6.6 ± 2.4 |

Figure 9B

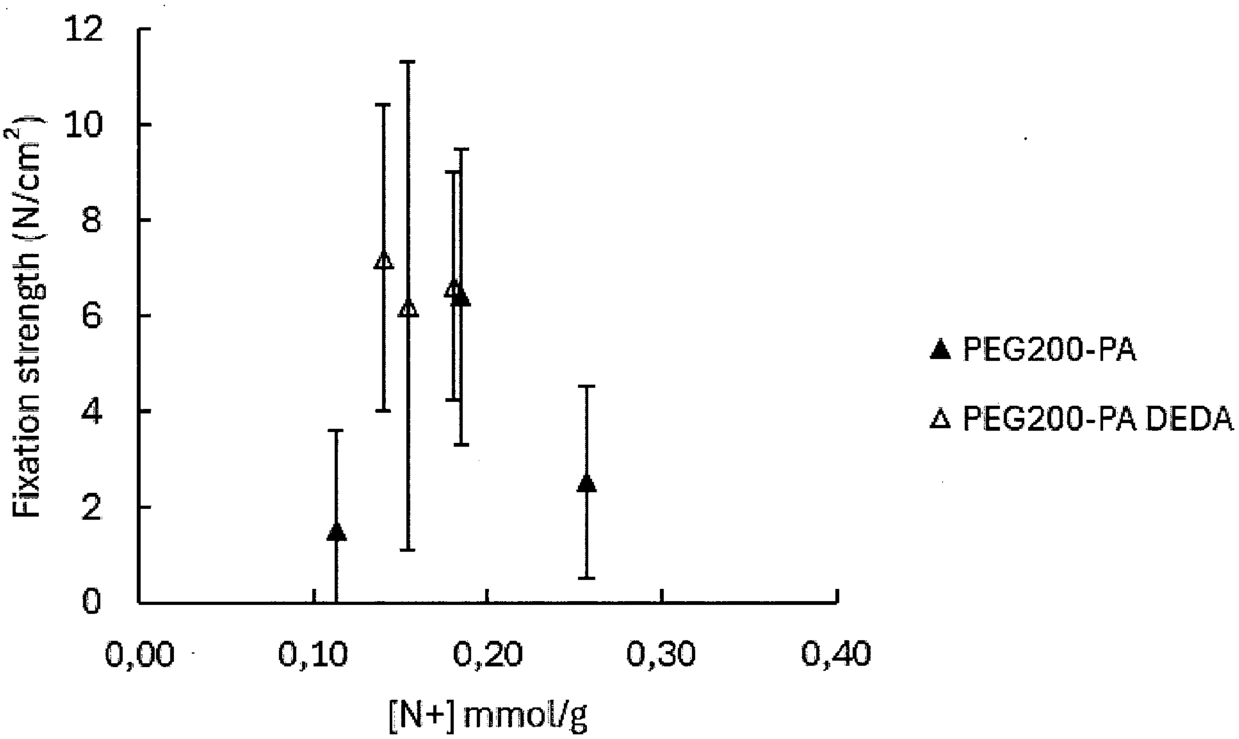
12
10
8
6
4
2
0
Fixation strength (N/cm2)
0,00
0,10
0,20
0,30
0,40
[N+] mmol/g
▲ PEG200-PA
△ PEG200-PA DEDA

**Figure 10**

| Example # | Diol | Diacid | Polyol | [A] (mmol/g) | [N+] (mmol/g) | Mn (g/mol) | Tensile strain (%) | Tensile strain wet24 (%) | Fixation strength (N/cm²) |
|---|---|---|---|---|---|---|---|---|---|
| **Example 34** | PEG 200 | Sebacic acid | Glycerol | 0.40 | 0.00 | 4528 | 100.3 ± 42.6 | 109.5 ± 12.8 | 0 ± 0 |
| **Example 23** | PEG 200 | Sebacic acid | Glycerol | 0.34 | 0.12 | 4803 | 75.0 ± 13.4 | 77.0 ± 14.6 | 1.4 ± 0.4 |
| **Example 15** | PEG 200 | Sebacic acid | Glycerol | 0.37 | 0.16 | 4295 | 70 ± 14 | 80 ± 8 | 8.1 ± 1.0 |
| **Example 20** | PEG 200 | Sebacic acid | Glycerol | 0.39 | 0.20 | 4128 | 73.5 ± 7.4 | 73.0 ± 8.3 | 8.9 ± 2.0 |
| **Example 22** | PEG 200 | Sebacic acid | Glycerol | 0.29 | 0.33 | 4709 | 116.2 ± 32.2 | 50.9 ± 9.9 | 5.9 ± 2.0 |

**Figure 11**

| Example # | Diol | Diacid | Polyol | [A] (mmol/g) | [N+] (mmol/g) | Mn (g/mol) | Young's Modulus (MPa) | Tensile strain (%) | Bending (cycles) |
|---|---|---|---|---|---|---|---|---|---|
| **Comparative polymer** | - | | | ~1 | ~0.7 | 1400-1700 | 5-6 | 10-20 | 10 ± 10 |
| **Example 35** | PEG 200 | Sebacic acid | Glycerol | 0,26 | 0,33 | 5561 | 0,48 ± 0.03 | 114,0 ± 27.1 | 39 ± 2 |
| **Example 36** | PEG 200 | Sebacic acid | Glycerol | 0,47 | 0,42 | 4452 | 1,51 ± 0.03 | 85,1 ± 24.3 | 40 ± 0 |
| **Example 37** | PEG 200 | Sebacic acid | Glycerol | 1,02 | 0,82 | 2576 | 3,76 ± 0.09 | 41,3 ± 2.7 | 38 ± 4 |
| **Example 38** | PEG 600 | Sebacic acid | Glycerol | 1,24 | 0,34 | 2691 | 9,78 ± 0.15 | 35,4 ± 4.8 | 38 ± 4 |

Europäisches Patentamt
European Patent Office
Office européen des brevets

# EUROPEAN SEARCH REPORT

Application Number
EP 24 31 5523

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 4 820 745 A (MUELLER HARTMUT [DE] ET AL) 11 April 1989 (1989-04-11)<br>* example F9 *<br>----- | 1-26 | INV.<br>C08G18/42<br>C08G18/81<br>C08G63/91 |
| A | US 2009/011486 A1 (BETTINGER CHRISTOPHER J [US] ET AL) 8 January 2009 (2009-01-08)<br>* paragraph [0006]; claims 1-2; examples 1-2 *<br>----- | 1-26 | |
| A | US 2022/380531 A1 (RHONÈ BENOIT [FR] ET AL) 1 December 2022 (2022-12-01)<br>* examples 5,8 *<br>----- | 1-26 | |

TECHNICAL FIELDS SEARCHED (IPC)

C09J
C08G

The present search report has been drawn up for all claims

| Place of search | Date of completion of the search | Examiner |
|---|---|---|
| The Hague | 8 July 2025 | Bezard, Stéphane |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C01)

# ANNEX TO THE EUROPEAN SEARCH REPORT ON EUROPEAN PATENT APPLICATION NO. EP 24 31 5523

This annex lists the patent family members relating to the patent documents cited in the above-mentioned European search report. The members are as contained in the European Patent Office EDP file on
The European Patent Office is in no way liable for these particulars which are merely given for the purpose of information.

08-07-2025

| Patent document cited in search report | Publication date | Patent family member(s) | Publication date |
|---|---|---|---|
| US 4820745 A | 11-04-1989 | US 4820745 A | 11-04-1989 |
| | | US 4822829 A | 18-04-1989 |
| US 2009011486 A1 | 08-01-2009 | NONE | |
| US 2022380531 A1 | 01-12-2022 | AU 2020370743 A1 | 26-05-2022 |
| | | BR 112022007788 A2 | 05-07-2022 |
| | | CA 3155938 A1 | 29-04-2021 |
| | | CN 114787236 A | 22-07-2022 |
| | | EP 4048717 A1 | 31-08-2022 |
| | | JP 7522187 B2 | 24-07-2024 |
| | | JP 2023505935 A | 14-02-2023 |
| | | JP 2024161354 A | 19-11-2024 |
| | | KR 20220094203 A | 05-07-2022 |
| | | US 2022380531 A1 | 01-12-2022 |
| | | WO 2021078962 A1 | 29-04-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 8143042 B
- US 20130231412 A
- US 7722894 B
- WO 2009067482 A
- WO 2014190302 A1
- WO 2021078962 A


### Non-patent literature cited in the description


- **ARTZI et al.** *Adv. Mater.*, 2009, vol. 21, 3399-3403
- **LANG et al.** A Blood-Resistant Surgical Glue for Minimally Invasive Repair of Vessels and Heart Defects. *Sci. Transl. Med.*, 08 January 2014, vol. 6 (218), 218-6
- **PATEL et al.** *Biomaterials*, vol. 34 (16), 3970-3983
- **N. LANG et al.** *Sci. Transl. Med.*, 2014, vol. 6, 218-6